(19) 【Europäisches Patentamt / European Patent Office / Office européen des brevets】

(11) **EP 4 775 595 A1**

(12) # EUROPEAN PATENT APPLICATION
published in accordance with Art. 153(4) EPC

(43) Date of publication:
**15.07.2026 Bulletin 2026/29**

(21) Application number: **24862067.6**

(22) Date of filing: **06.09.2024**

(51) International Patent Classification (IPC):
**C07K 16/28** (2006.01)   **A61K 39/395** (2006.01)
**A61P 27/02** (2006.01)   **A61P 37/06** (2006.01)

(52) Cooperative Patent Classification (CPC):
**A61K 39/395; A61P 27/02; A61P 37/06;
C07K 16/28**

(86) International application number:
**PCT/CN2024/117376**

(87) International publication number:
**WO 2025/051229 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **08.09.2023  CN 202311157087**

(71) Applicant: **Beijing Tuo Jie Biopharmaceutical Co.
Ltd.
Beijing 102206 (CN)**

(72) Inventors:
• **XU, Jinjing
  Beijing 102206 (CN)**

• **WANG, Lei
  Beijing 102206 (CN)**
• **ZHAN, Yikang
  Beijing 102206 (CN)**
• **LIU, Xiao
  Beijing 102206 (CN)**

(74) Representative: **Dragotti & Associati S.P.A.
Via Nino Bixio, 7
20129 Milano (MI) (IT)**

Remarks:
The complete document including Reference
Table(s) and the Sequence Listing(s) can be
downloaded from the EPO website

(54)  **IGF-1R AND TSHR BINDING PROTEIN AND PHARMACEUTICAL USE THEREOF**

(57)   Provided are an IGF-1R and TSHR binding protein and a pharmaceutical use thereof. Specifically, provided are an IGF-1R/TSHR binding protein, an IGF-1R binding protein, a TSHR binding protein, a method for using same for treatment of an autoimmune disease (e.g. Graves' orbitopathy), and a related pharmaceutical use.

EP 4 775 595 A1

## Description

The present application claims priority to Chinese Patent Application No. CN202311157087.9 filed on September 8, 2023.

## TECHNICAL FIELD

The present disclosure relates to an IGF-1R/TSHR-binding protein, an IGF-1R-binding protein, a TSHR-binding protein, a coding nucleic acid therefor, a pharmaceutical composition thereof, and methods for treating autoimmune diseases (e.g., Graves' orbitopathy) by using same and related pharmaceutical use thereof.

## BACKGROUND

Graves' orbitopathy (GO), also known as thyroid-associated ophthalmopathy (TAO) or thyroid eye disease (TED), is an organ-specific autoimmune disease that is associated with and relatively independent of Graves' disease. In patients, the immune system overstimulates the thyroid, causing the thyroid to secrete large amounts of thyroid hormones. Orbital fibroblasts (OFs) express thyroid-stimulating hormone receptor (TSHR), insulin-like growth factor-1 receptor (IGF-1R), and immunomodulatory molecules. Upon appropriate stimulation, some of these cells may produce hyaluronic acid (HA), inflammatory mediators, and autoantibodies, while some of these cells may differentiate into adipocytes. OFs are the main target cells of autoimmunity in Graves' orbitopathy (Terry J. Smith, N Engl J Med 375; 16 (2016)). Adipocytes differentiated from OFs are the major source of TSHR in the orbital tissues of patients with GO. Studies have demonstrated that TSHR mRNA and protein are expressed in orbital adipose tissues of Graves' disease patients with or without orbitopathy, and the TSHR level in patients with GO is indeed higher than that in patients without GO, suggesting that the increased expression of TSHR in the orbit may be involved in the development of the disease (Wang Y, Invest Ophthalmol Vis Sci, 55; 3 (2014)). Studies have found that IGF-1R and TSHR are expressed at the same cellular location and synergistically form a functional complex, which activates B cells to produce thyroid-stimulating antibodies capable of binding to TSHR. TSHR and IGF-1R jointly mediate the activation of downstream signaling pathways, causing OFs to produce a large number of inflammatory factors, such as IL-8, prostaglandin E2, and monocyte chemoattractant protein. Meanwhile, after the TSHR is activated, the OFs secrete a large amount of hyaluronic acid, resulting in dilation, edema, and inflammation of the retrobulbar adipose connective tissue. In addition, binding of IGF-1 to IGF-1R can further promote massive proliferation of OFs.

Tepezza is currently the only globally approved antibody drug targeting IGF-1R and has not yet been approved in China. K1-70 is an inhibitory antibody against TSHR and is currently in Phase I clinical trials. The phase I results have shown that it can effectively alleviate symptoms associated with GO patients (Jadwiga Furmaniak, Clinical Endocrinology, 96; 878-887 (2022)).

A single-domain antibody has a molecular weight of only 12-15 kDa and contains 3 CDRs. It is currently the smallest known antibody with complete antigen-binding activity, exhibiting multiple advantages such as high binding capacity, high specificity, ease of engineering, high solubility, high stability, and high expression levels. The present disclosure provides an anti-TSHR single-domain antibody with a novel sequence structure and an anti-IGF-1R single-domain antibody with a novel sequence structure, and constructs a bispecific antibody of the two. Testing has confirmed that the bispecific antibody achieves a synergistic effect, demonstrating potential to serve as a more efficacious and safer therapeutic agent for the clinical treatment of Graves' orbitopathy.

## SUMMARY

An IGF-1R/TSHR-binding protein, an IGF-1R-binding protein, a TSHR-binding protein, a coding nucleic acid therefor, a pharmaceutical composition thereof, and methods for treating diseases by using same and related pharmaceutical use thereof are provided.

IGF-1R-Binding Protein

The present disclosure provides an IGF-1R-binding protein, comprising an immunoglobulin single variable domain, wherein the immunoglobulin single variable domain comprises:

1) a CDR1, a CDR2, and a CDR3 from the amino acid sequence set forth in any one of SEQ ID NOs: 4, 20-24, and 34, or 2) a CDR1, a CDR2, and a CDR3 from the amino acid sequence set forth in any one of SEQ ID NOs: 5 and 25-30, wherein the CDR1, the CDR2, and the CDR3 are numbered according to the Kabat, IMGT, Chothia, AbM, or Contact numbering scheme, for example, according to the Kabat numbering scheme.

In some embodiments, provided is an IGF-1R-binding protein, comprising any one of or any combination of the CDR1, the CDR2, and the CDR3 described above.

In some embodiments, provided is an IGF-1R-binding protein, comprising an immunoglobulin single variable domain, wherein the amino acid sequences of a CDR1, a CDR2, and a CDR3 of the immunoglobulin single variable domain are set forth in:

1) SEQ ID NOs: 7, 8, and 35, respectively, SEQ ID NOs: 7, 8, and 9, respectively, or SEQ ID NOs: 7, 8, and 36, respectively;
2) SEQ ID NOs: 10, 11, and 12, respectively.

In some embodiments, the immunoglobulin single variable domain in the aforementioned IGF-IR-binding protein is engineered by humanization, back mutation, affinity maturation, T cell epitope (TCE) removal/reduction, reduction of antibody deamidation, and/or reduction of antibody isomerization.

In some embodiments, the immunoglobulin single variable domain is obtained by TCE removal/reduction and has one or more changes in one or more CDRs, and the changes cause a reduction in the immunogenicity of the IGF-1R-binding protein.

In some embodiments, heavy chain framework regions of a human germline template used for humanizing the immunoglobulin single variable domain in the aforementioned IGF-1R-binding protein are derived from IGHV3-23, IGHV3-66, or IGHJ4. For example, FR1, FR2, and FR3 are derived from IGHV3-66, and FR4 is derived from IGHJ4.

In some embodiments, when the aforementioned immunoglobulin single variable domain comprises a CDR1, a CDR2, and a CDR3 set forth in SEQ ID NOs: 7, 8, and 35, respectively, SEQ ID NOs: 7, 8, and 9, respectively, or SEQ ID NOs: 7, 8, and 36, respectively, the immunoglobulin single variable domain comprises one, more, or any combination of mutations in the amino acid residues at positions 23, 27, 30, 37, 44, 45, 47, 74, 82, and 94 (numbered according to the Kabat numbering scheme), e.g., one, more, or any combination of mutations selected from the group consisting of 23T, 27L, 30G, 37F, 44E, 45R, 47G, 74A, 82D or A, and 94V.

In some embodiments, when the aforementioned immunoglobulin single variable domain comprises a CDR1, a CDR2, and a CDR3 set forth in SEQ ID NOs: 10, 11, and 12, respectively, the immunoglobulin single variable domain comprises one, more, or any combination of mutations in the amino acid residues at positions 26, 27, 28, 44, 45, 47, 48, 49, 73, 74, 77, 94, and 103 (numbered according to the Kabat numbering scheme), e.g., one, more, or any combination of mutations selected from the group consisting of 26R, 27Y, 28I, 44E, 45R, 47E or G, 48I, 49A, 73G, 74A, 77F, 94A, and 103L.

In some embodiments, the amino acid sequence of the immunoglobulin single variable domain in the aforementioned IGF-1R-binding protein is set forth in:

1) any one of SEQ ID NOs: 4, 20-24, and 34, or has at least 80% (preferably at least 90%) sequence identity to any one of SEQ ID NOs: 4, 20-24, and 34;
2) any one of SEQ ID NOs: 5 and 25-30, or has at least 80% (preferably at least 90%) sequence identity to any one of SEQ ID NOs: 5 and 25-30.

In the present disclosure, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity.

"At least 90% (sequence) identity" encompasses at least 90%, at least 91%, at least 92%, at least 93%, at least 94%, at least 95%, at least 96%, at least 97%, at least 98%, at least 99%, or 100% (sequence) identity.

In some embodiments, the aforementioned IGF-1R-binding protein comprises or is an antibody or an antigen-binding fragment thereof that specifically binds to a human IGF-1R protein or a fragment thereof. In some specific embodiments, the antibody or the antigen-binding fragment thereof is, for example, a camelid antibody, a chimeric antibody, a humanized antibody, or a fully human antibody, or an antigen-binding fragment thereof. In some specific embodiments, the antibody or the antigen-binding fragment thereof is a recombinant antibody or a fragment thereof. In some specific embodiments, the antibody or the antigen-binding fragment thereof is a linear antibody, a single-chain antibody, a nanobody, a peptibody, a domain antibody, a diabody, a triabody, a tetrabody, a tandem di-scFv, or a tandem tri-scFv. In some specific embodiments, the antibody is a monospecific antibody, a bispecific antibody, or a multispecific antibody (e.g., a trispecific antibody).

In some embodiments, the immunoglobulin single variable domain in the aforementioned IGF-1R-binding protein is a single-domain antibody or VHH, or the IGF-1R-binding protein is a single-domain antibody or VHH.

In some embodiments, the present disclosure provides an IGF-1R-binding protein, comprising one or more (e.g., 2, 3, 4, 5, 6, 7, or 8) of the aforementioned immunoglobulin single variable domains, wherein the immunoglobulin single variable domains may be identical or different and may form a dimeric or multimeric molecule.

In some embodiments, the aforementioned IGF-1R-binding protein further comprises an Fc region of a human immunoglobulin; for example, the Fc region is an Fc region of human IgG1, IgG2, IgG3, or IgG4. In some embodiments, the Fc region is an Fc region of human IgG1; for example, the Fc region is set forth in SEQ ID NO: 16 or has at least 80% (preferably at least 90%) sequence identity thereto. In some embodiments, the Fc region has increased stability or comprises a mutation that makes the Fc region more stable than a wild-type Fc region. In some embodiments, the Fc region can cause the binding protein to form a dimeric molecule. In some specific embodiments, the Fc region comprises a mutation that extends the *in vivo* half-life, which depends on the FcRn binding affinity. The extension of half-life can allow for a decrease in the amount of a drug given to a patient and/or a reduction in the frequency of administration. For example, the Fc region comprises an M252Y, S254T, and/or T256E mutation. In some specific embodiments, the Fc region may be an Fc region with a reduced effector function; for example, the Fc region may comprise a mutation, and exemplary IgG Fc regions with a reduced effector function comprise the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1).

In some embodiments, in the aforementioned IGF-1R-binding protein, the immunoglobulin single variable domain and the Fc region are linked, either directly or by a linker. The linker may be a non-functional amino acid sequence having a length of 1-20 or more amino acids and lacking a secondary or higher-order structure. For example, the linker is a flexible linker, such as $G_4S$ (SEQ ID NO: 53), GS, GAP, $(G_4S)_2$ (SEQ ID NO: 54), $(G_4S)_3$ (SEQ ID NO: 55), $(G_4S)_4$ (SEQ ID NO: 56), $(G_4S)_5$ (SEQ ID NO: 57), or ASGS (SEQ ID NO: 58), e.g., $(G_4S)_2$; for example, the linker is represented by $(G_mS_n)_h$ or $(G_mQ_n)_h$ or $(GGNGT)_h$ (SEQ ID NO: 59) or $(YGNGT)_h$ (SEQ ID NO: 60) or $(EPKSS)_h$ (SEQ ID NO: 61), wherein m and n are each independently selected from the group consisting of integers of 1-8, and h is independently selected from the group consisting of integers of 1-20.

In some embodiments, the IGF-1R-binding protein of the present disclosure is an anti-IGF-1R antibody or an antigen-binding fragment thereof, or a conjugate or fusion protein comprising the antibody or the antigen-binding fragment.

In some embodiments, provided is an IGF-1R-binding protein, comprising the amino acid sequence set forth in any one of SEQ ID NOs: 17 or 37 and 18 or an amino acid sequence having at least 80% (preferably at least 90%) identity to any one of SEQ ID NOs: 17 or 37 and 18.

In some embodiments, the aforementioned IGF-1R-binding protein has a function or property selected from the group consisting of at least one of the following:

(a) Binding to human IGF-1R or an epitope thereof with a $K_D$ value (M) of $\leq 10^{-7}$. The method for determining the $K_D$ value is conventional in the art, for example, the method in Example 3 of the present disclosure. In some embodiments, the single-domain antibody of the present disclosure binds to human IGF-1R or an epitope thereof with a $K_D$ value of $\leq 10^{-10}$, or the single-domain antibody of the present disclosure binds to human IGF-1R or an epitope thereof with a $K_D$ value of $\leq 10^{-8}$ or $\leq 10^{-9}$.

(b) Inhibition of IGF-1R downstream signaling pathways, such as pAKT. In some embodiments, the $IC_{50}$ (nM) for inhibition of pAKT in A549 cells is less than 10, less than 5, less than 2, less than 1, less than 0.5, or less than 0.2. In some embodiments, the $IC_{50}$ value is determined by the method in Example 4 of the present disclosure.

In some embodiments, the aforementioned IGF-1R-binding protein of the present disclosure may bind to IGF-1R with a $K_D$ value of $\leq 1 \times 10^{-7}$ M, e.g., $\leq 1 \times 10^{-8}$ M, or $\leq 1 \times 10^{-9}$ M, or $\leq 1 \times 10^{-10}$ M.

In some embodiments, the aforementioned IGF-1R-binding protein of the present disclosure encompasses a variant, wherein the variant comprises one or more (e.g., 1, 2, 3, 4, 5, 6, 7, 8, 9, or 10) amino acid mutations as compared to any one of SEQ ID NOs: 4, 20-24, and 34 or any one of SEQ ID NOs: 5 and 25-30; the amino acid mutations may be conservative replacements, substitutions, or modifications, and/or deletions or additions that do not affect function; the amino acid mutations may occur in the CDRs and/or FRs.

In some embodiments, provided is an anti-IGF-1R antibody or an antigen-binding fragment thereof that binds to or competes for binding to the same epitope with the immunoglobulin single variable domain in the aforementioned IGF-1R-binding protein of the present disclosure.

In some embodiments, provided is an anti-IGF-1R antibody or an antigen-binding fragment thereof that blocks the binding of the immunoglobulin single variable domain in the aforementioned IGF-1R-binding protein of the present disclosure to IGF-1R (e.g., human IGF-1R).

In some embodiments, provided is an anti-IGF-IR antibody or an antigen-binding fragment thereof whose binding to IGF-1R (e.g., human IGF-1R) is blocked by the immunoglobulin single variable domain in the aforementioned IGF-1R-binding protein of the present disclosure.

In some embodiments, provided is a protein or molecule, comprising any one or more (e.g., 1, 2, 3, 4, 5, 6, 7, 8, 9, or 10) of the immunoglobulin single variable domains in the aforementioned IGF-1R-binding proteins of the present disclosure, wherein the immunoglobulin single variable domains are identical or different. For example, the protein or molecule is a conjugate, and the conjugate may, for example, comprise any detectable label.

TSHR-Binding Protein

The present disclosure provides a TSHR-binding protein, comprising an immunoglobulin single variable domain.

In some embodiments, the immunoglobulin single variable domain in the TSHR-binding protein comprises a CDR1, a CDR2, and a CDR3 from the amino acid sequence set forth in any one of SEQ ID NOs: 6 and 31-33, wherein the CDR1, the CDR2, and the CDR3 are numbered according to the Kabat, IMGT, Chothia, AbM, or Contact numbering scheme, for example, according to the Kabat numbering scheme.

In some embodiments, provided is a TSHR-binding protein, comprising any one of or any combination of the CDR1, the CDR2, and the CDR3 described above.

In some embodiments, provided is a TSHR-binding protein, comprising an immunoglobulin single variable domain, wherein the amino acid sequences of a CDR1, a CDR2, and a CDR3 of the immunoglobulin single variable domain are set forth in SEQ ID NOs: 13-15, respectively.

In some embodiments, the immunoglobulin single variable domain in the aforementioned TSHR-binding protein is engineered by humanization, back mutation, affinity maturation, T cell epitope (TCE) removal/reduction, reduction of antibody deamidation, and/or reduction of antibody isomerization.

In some embodiments, the immunoglobulin single variable domain is obtained by TCE removal/reduction and has one or more changes in one or more CDRs, and the changes cause a reduction in the immunogenicity of the TSHR-binding protein.

In some embodiments, heavy chain framework regions of a human germline template used for humanizing the immunoglobulin single variable domain in the aforementioned TSHR-binding protein are derived from IGHV3-23, IGHV3-66, or IGHJ4. For example, FR1, FR2, and FR3 are derived from IGHV3-23, and FR4 is derived from IGHJ4.

In some embodiments, the immunoglobulin single variable domain comprises one, more, or any combination of mutations in the amino acid residues at positions 24, 25, 27, 29, 37, 44, 45, 46, 47, 49, 71, 74, 93, and 94 (numbered according to the Kabat numbering scheme), e.g., one, more, or any combination of mutations selected from the group consisting of 24G, 25P, 27Y, 29S, 37F, 44E, 45R, 46T, 47G, 49A, 71Q, 74A, 93W, and 94A.

In some embodiments, the amino acid sequence of the immunoglobulin single variable domain in the aforementioned TSHR-binding protein is set forth in any one of SEQ ID NOs: 6 and 31-33 or has at least 80% (preferably at least 90%) sequence identity to any one of SEQ ID NOs: 6 and 31-33.

In some embodiments, the aforementioned TSHR-binding protein comprises or is an antibody or an antigen-binding fragment thereof that specifically binds to a human TSHR protein or a fragment thereof. In some specific embodiments, the antibody or the antigen-binding fragment thereof is, for example, a camelid antibody, a chimeric antibody, a humanized antibody, or a fully human antibody, or an antigen-binding fragment thereof. In some specific embodiments, the antibody or the antigen-binding fragment thereof is a recombinant antibody or a fragment thereof. In some specific embodiments, the antibody or the antigen-binding fragment thereof is a linear antibody, a single-chain antibody, a nanobody, a peptibody, a domain antibody, a diabody, a triabody, a tetrabody, a tandem di-scFv, or a tandem tri-scFv. In some specific embodiments, the antibody is a monospecific antibody, a bispecific antibody, or a multispecific antibody (e.g., a trispecific antibody).

In some embodiments, the immunoglobulin single variable domain in the aforementioned TSHR-binding protein is a single-domain antibody or VHH, or the TSHR-binding protein is a single-domain antibody or VHH.

In some embodiments, the present disclosure provides a TSHR-binding protein, comprising one or more (e.g., 2, 3, 4, 5, 6, 7, or 8) of the aforementioned immunoglobulin single variable domains, wherein the immunoglobulin single variable domains may be identical or different and may form a dimeric or multimeric molecule.

In some embodiments, the aforementioned TSHR-binding protein further comprises an Fc region of a human immunoglobulin; for example, the Fc region is an Fc region of human IgG1, IgG2, IgG3, or IgG4. In some embodiments, the Fc region is an Fc region of human IgG1; for example, the Fc region is set forth in SEQ ID NO: 16 or has at least 80% (preferably at least 90%) sequence identity thereto. In some embodiments, the Fc region has increased stability or comprises a mutation that makes the Fc region more stable than a wild-type Fc region. In some embodiments, the Fc region can cause the binding protein to form a dimeric molecule. In some specific embodiments, the Fc region comprises a mutation that extends the *in vivo* half-life, which depends on the FcRn binding affinity. The extension of half-life can allow for a decrease in the amount of a drug given to a patient and/or a reduction in the frequency of administration. For example, the Fc region comprises an M252Y, S254T, and/or T256E mutation. In some specific embodiments, the Fc region may be an Fc region with a reduced effector function; for example, the Fc region may comprise a mutation, and exemplary IgG Fc regions with a reduced effector function comprise the following substitutions: N297A or N297Q (IgG1); L234A/L235A (IgG1); V234A/G237A (IgG2); L235A/G237A/E318A (IgG4); H268Q/V309L/A330S/A331S (IgG2);

C220S/C226S/C229S/P238S (IgG1); C226S/C229S/E233P/L234V/L235A (IgG1); L234F/L235E/P331S (IgG1); or S267E/L328F (IgG1).

In some embodiments, in the aforementioned TSHR-binding protein, the immunoglobulin single variable domain and the Fc region are linked, either directly or by a linker. The linker may be a non-functional amino acid sequence having a length of 1-20 or more amino acids and lacking a secondary or higher-order structure. For example, the linker is a flexible linker, such as $G_4S$, GS, GAP, $(G_4S)_2$, $(G_4S)_3$, $(G_4S)_4$, $(G_4S)_5$, or ASGS; in a further example, the linker is $(G_4S)_2$; for example, the linker is represented by $(G_mS_n)_h$ or $(G_mQ_n)_h$ or $(GGNGT)_h$ or $(YGNGT)_h$ or $(EPKSS)_h$, wherein m and n are each independently selected from the group consisting of integers of 1-8, and h is independently selected from the group consisting of integers of 1-20.

In some embodiments, the TSHR-binding protein of the present disclosure is an anti-TSHR antibody or an antigen-binding fragment thereof, or a conjugate or fusion protein comprising the antibody or the antigen-binding fragment.

In some embodiments, provided is a TSHR-binding protein, comprising the amino acid sequence set forth in any one of SEQ ID NOs: 19 and 38 or an amino acid sequence having at least 80% (preferably at least 90%) identity to any one of SEQ ID NOs: 19 and 38.

In some embodiments, the aforementioned TSHR-binding protein has a function or property selected from the group consisting of at least one of the following:

(a) Binding to human TSHR or an epitope thereof with a $K_D$ value (M) of $\leq 10^{-7}$. The method for determining the $K_D$ value is conventional in the art, for example, the method in Example 3 of the present disclosure.
(b) Inhibition of cAMP signals. In some embodiments, the $IC_{50}$ (nM) for inhibition of cAMP in HEK293 cells is less than 20, less than 10, less than 5, or less than 3. In some embodiments, the $IC_{50}$ value is determined by the method in Example 5 of the present disclosure.
(c) Inhibition of serum total thyroxine T4 and/or free T4. In some embodiments, T4 is detected according to the method in Example 6 of the present disclosure.

In some embodiments, the aforementioned TSHR-binding protein of the present disclosure may bind to TSHR with a $K_D$ value of $\leq 1 \times 10^{-8}$ M, e.g., $\leq 1 \times 10^{-9}$ M, or $\leq 1 \times 10^{-10}$ M, or $\leq 1 \times 10^{-11}$ M, or $\leq 1 \times 10^{-12}$ M.

In some embodiments, the aforementioned TSHR-binding protein of the present disclosure encompasses a variant, wherein the variant comprises one or more (e.g., 1, 2, 3, 4, 5, 6, 7, 8, 9, or 10) amino acid mutations as compared to any one of SEQ ID NOs: 6 and 31-33; the amino acid mutations may be conservative replacements, substitutions, or modifications, and/or deletions or additions that do not affect function; the amino acid mutations may occur in the CDRs and/or FRs.

In some embodiments, provided is an anti-TSHR antibody or an antigen-binding fragment thereof that binds to or competes for binding to the same epitope with the immunoglobulin single variable domain in the aforementioned TSHR-binding protein of the present disclosure.

In some embodiments, provided is an anti-TSHR antibody or an antigen-binding fragment thereof that blocks the binding of the immunoglobulin single variable domain in the aforementioned TSHR-binding protein of the present disclosure to TSHR (e.g., human TSHR).

In some embodiments, provided is an anti-TSHR antibody or an antigen-binding fragment thereof whose binding to TSHR (e.g., human TSHR) is blocked by the immunoglobulin single variable domain in the aforementioned TSHR-binding protein of the present disclosure.

In some embodiments, provided is a protein or molecule, comprising any one or more (e.g., 1, 2, 3, 4, 5, 6, 7, 8, 9, or 10) of the immunoglobulin single variable domains in the aforementioned TSHR-binding proteins of the present disclosure, wherein the immunoglobulin single variable domains are identical or different. For example, the protein or molecule is a conjugate, and the conjugate may, for example, comprise any detectable label.

Combination of IGF-1R-Binding Protein and TSHR-Binding Protein

The present disclosure provides a combination or combined use of an IGF-1R-binding protein and a TSHR-binding protein.

In some embodiments, the IGF-1R-binding protein is any of the aforementioned IGF-1R-binding proteins of the present disclosure, and the TSHR-binding protein is any of the aforementioned TSHR-binding proteins of the present disclosure.

In some embodiments, in the combination of the present disclosure, the IGF-1R-binding protein and the TSHR-binding protein are not covalently linked.

In some specific embodiments, the IGF-1R-binding protein comprises the amino acid sequence set forth in any one of SEQ ID NOs: 4, 20-24, and 34 or an amino acid sequence having at least 80% (preferably at least 90%) identity to any one of SEQ ID NOs: 4, 20-24, and 34, or comprises the amino acid sequence set forth in any one of SEQ ID NOs: 5 and 25-30 or an amino acid sequence having at least 80% (preferably at least 90%) identity to any one of SEQ ID NOs: 5 and 25-30.

In some specific embodiments, the TSHR-binding protein comprises the amino acid sequence set forth in any one of SEQ ID NOs: 6 and 31-33 or an amino acid sequence having at least 80% (preferably at least 90%) identity to any one of SEQ ID NOs: 6 and 31-33.

In some specific embodiments, the IGF-1R-binding protein comprises the amino acid sequence set forth in any one of SEQ ID NOs: 17 or 37 and 18 or an amino acid sequence having at least 80% (preferably at least 90%) identity to any one of SEQ ID NOs: 17 or 37 and 18, and the TSHR-binding protein comprises the amino acid sequence set forth in any one of SEQ ID NOs: 19 and 38 or an amino acid sequence having at least 80% (preferably at least 90%) identity to any one of SEQ ID NOs: 19 and 38.

In some specific embodiments, the IGF-1R-binding protein comprises the amino acid sequence set forth in SEQ ID NO: 34, and the TSHR-binding protein comprises the amino acid sequence set forth in SEQ ID NO: 38.

In some specific embodiments, the IGF-1R-binding protein comprises the amino acid sequence set forth in SEQ ID NO: 37, and the TSHR-binding protein comprises the amino acid sequence set forth in SEQ ID NO: 33.

In some embodiments, the combination of the IGF-1R-binding protein and the TSHR-binding protein has the following functions or properties:

(a) synergistic inhibition of pERK, wherein in some embodiments, pERK is detected according to the method in Example 9 of the present disclosure; and/or
(b) synergistic inhibition of hyaluronic acid secretion by cells (e.g., human orbital fibroblasts). In some embodiments, the inhibition of the secretion of hyaluronic acid by cells is detected according to the method in Example 10 of the present disclosure.

In some embodiments, the combination of the IGF-1R-binding protein and the TSHR-binding protein of the present disclosure is prepared in the form of a kit, wherein the IGF-1R-binding protein and the TSHR-binding protein are provided in separate containers or in the same container.

Administration of IGF-1R-Binding Protein in Combination with TSHR-Binding Protein

In some embodiments, the present disclosure provides any one of the aforementioned IGF-1R-binding proteins for use in treating a disease (as an example, Graves' orbitopathy), wherein the IGF-1R-binding protein is administered in combination with the TSHR-binding protein of the present disclosure.

In some other embodiments, the present disclosure provides any one of the aforementioned TSHR-binding proteins for use in treating a disease (as an example, Graves' orbitopathy), wherein the TSHR-binding protein is administered in combination with the IGF-1R-binding protein of the present disclosure.

In some embodiments, the present disclosure provides use of any one of the aforementioned TSHR-binding proteins in combination with any one of the aforementioned IGF-1R-binding proteins in the manufacture of a medicament for treating or preventing a disease (as an example, Graves' orbitopathy).

In some embodiments, the present disclosure provides a method for treating or preventing a disease (as an example, Graves' orbitopathy), comprising administering to a patient any one of the aforementioned TSHR-binding proteins and any one of the aforementioned IGF-1R-binding proteins.

IGF-1R/TSHR-Binding Protein

The present disclosure provides an IGF-1R/TSHR-binding protein that is capable of binding to IGF-1R (or an epitope thereof) and TSHR (or an epitope thereof) simultaneously or sequentially.

In some embodiments, the IGF-1R/TSHR-binding protein comprises a first antigen-binding domain that specifically binds to TSHR and a second antigen-binding domain that specifically binds to IGF-1R.

In some embodiments, the first antigen-binding domain that specifically binds to TSHR comprises an immunoglobulin single variable domain, and the immunoglobulin single variable domain in the first antigen-binding domain comprises a CDR1, a CDR2, and a CDR3 from the amino acid sequence set forth in any one of SEQ ID NOs: 6 and 31-33. In some embodiments, the second antigen-binding domain that specifically binds to IGF-1R comprises an immunoglobulin single variable domain, and the immunoglobulin single variable domain in the second antigen-binding domain comprises: 1) a CDR1, a CDR2, and a CDR3 from the amino acid sequence set forth in any one of SEQ ID NOs: 4, 20-24, and 34, or 2) a CDR1, a CDR2, and a CDR3 from the amino acid sequence set forth in any one of SEQ ID NOs: 5 and 25-30.

The aforementioned CDR1, CDR2, and CDR3 are numbered according to the Kabat, IMGT, Chothia, AbM, or Contact numbering scheme, for example, according to the Kabat numbering scheme.

In some embodiments, provided is an IGF-1R/TSHR-binding protein, comprising any one of or any combination of the CDR1, the CDR2, and the CDR3 described above.

In some embodiments, the amino acid sequences of the CDR1, the CDR2, and the CDR3 of the immunoglobulin single variable domain in the first antigen-binding domain that specifically binds to TSHR are set forth in SEQ ID NOs: 13-15, respectively.

In some embodiments, the amino acid sequences of the CDR1, the CDR2, and the CDR3 of the immunoglobulin single variable domain in the second antigen-binding domain that specifically binds to IGF-1R are set forth in:

1) SEQ ID NOs: 7, 8, and 35, respectively, SEQ ID NOs: 7, 8, and 9, respectively, or SEQ ID NOs: 7, 8, and 36, respectively;

2) SEQ ID NOs: 10, 11, and 12, respectively.

In some embodiments, the immunoglobulin single variable domain in the first antigen-binding domain that specifically binds to TSHR and/or the immunoglobulin single variable domain in the second antigen-binding domain that specifically binds to IGF-1R is/are engineered by humanization, back mutation, affinity maturation, T cell epitope (TCE) removal/reduction, reduction of antibody deamidation, and/or reduction of antibody isomerization. The engineering of TCE removal/reduction may cause the immunoglobulin single variable domain to have one or more changes in one or more CDRs, resulting in reduced immunogenicity.

In some embodiments, heavy chain framework regions of a human germline template used for humanization are derived from IGHV3-23, IGHV3-66, or IGHJ4. For example, FR1, FR2, and FR3 are derived from IGHV3-23 or IGHV3-66, and FR4 is derived from IGHJ4.

In some embodiments, the amino acid sequence of the immunoglobulin single variable domain in the aforementioned first antigen-binding domain that specifically binds to TSHR is set forth in any one of SEQ ID NOs: 6 and 31-33 or has at least 80% (preferably at least 90%) sequence identity to any one of SEQ ID NOs: 6 and 31-33.

In some embodiments, the amino acid sequence of the immunoglobulin single variable domain in the aforementioned second antigen-binding domain that specifically binds to IGF-1R is set forth in any one of SEQ ID NOs: 4, 20-24, and 34 or has at least 80% (preferably at least 90%) sequence identity to any one of SEQ ID NOs: 4, 20-24, and 34, or is set forth in any one of SEQ ID NOs: 5 and 25-30 or has at least 80% (preferably at least 90%) sequence identity to any one of SEQ ID NOs: 5 and 25-30.

In some embodiments, the aforementioned IGF-1R/TSHR-binding protein further comprises an Fc region of a human immunoglobulin; for example, the Fc region is an Fc region of human IgG1, IgG2, IgG3, or IgG4. In some embodiments, the Fc region is an Fc region of human IgG1; for example, the Fc region is set forth in SEQ ID NO: 16 or has at least 80% (preferably at least 90%) sequence identity thereto. In some embodiments, the Fc region has increased stability or comprises a mutation that makes the Fc region more stable than a wild-type Fc region. In some specific embodiments, the Fc region comprises a mutation that extends the *in vivo* half-life, which depends on the FcRn binding affinity. The extension of half-life can allow for a decrease in the amount of a drug given to a patient and/or a reduction in the frequency of administration. For example, the Fc region comprises an M252Y, S254T, and/or T256E mutation. In some specific embodiments, the Fc region may be an Fc region with a reduced effector function; for example, the Fc region may comprise a mutation, and exemplary IgG Fc regions with a reduced effector function comprise the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1). In some embodiments, the Fc region can cause the binding protein to form a dimeric molecule.

In some embodiments, the Fc region comprises a first subunit (Fc1) and a second subunit (Fc2), and a mutation that pairs the two subunits (Fc1 and Fc2) of the Fc region to form a dimer or a mutation that reduces homodimerization is introduced. In some embodiments, the Fc region comprises a knob-into-hole mutation that promotes the association of the first subunit and the second subunit. For example, within the CH3/CH3 interface, one, two, or more amino acid residues in the CH3 domain of Fc1 are mutated with one or more amino acid residues having a larger side-chain volume, thereby forming protuberances (or knobs) in the surface of the CH3 domain of Fc1; one, two, or more amino acid residues in the CH3 domain of Fc2 that interact with the CH3 domain of Fc1 are mutated with amino acid residues having a smaller side-chain volume, thereby forming cavities (or holes) in the surface of the CH3 domain of Fc2 that interacts with the CH3 domain of Fc1. In some specific embodiments, the Fc1 comprises one or more amino acid substitutions at positions selected from the group consisting of 354, 356, 358, and 366, and the Fc2 comprises one or more amino acid substitutions at positions selected from the group consisting of 349, 356, 358, 366, 368, and 407. In some specific embodiments, the Fc1 comprises a mutation at position 366, and the Fc2 comprises a mutation selected from the group consisting of a mutation at position 366, a mutation at position 368, and a mutation at position 407, or any combination thereof. In some specific embodiments, the Fc1 comprises a mutation at position 354 or 356, and the Fc2 comprises a mutation at position 349. In some specific embodiments, the Fc1 comprises a mutation at position 354 or 356, and the Fc2 comprises mutations at positions 349, 366, 368, and 407.

In some specific embodiments, the Fc1 comprises one or more amino acid substitutions selected from the group consisting of 354C, 356E, 358M, and 366W, and the Fc2 comprises one or more amino acid substitutions selected from the group consisting of 349C, 356E, 358M, 366S, 368A, and 407V. In some specific embodiments, the Fc1 comprises a 366W mutation, and the Fc2 comprises a mutation selected from the group consisting of 366S, 368A, and 407V, or any combination thereof. In some specific embodiments, the Fc1 comprises a 354C or 356C mutation, and the Fc2 comprises a 349C mutation. Alternatively, in some specific embodiments, the Fc1 comprises 354C/366W mutations, and the Fc2 comprises 349C/366S/368A/407V mutations.

In some specific embodiments, the aforementioned IGF-1R/TSHR-binding protein further comprises a linker. For example, the immunoglobulin single variable domain in the first antigen-binding domain that specifically binds to TSHR and/or the immunoglobulin single variable domain in the second antigen-binding domain that specifically binds to IGF-1R is/are linked to the Fc region via a linker. The linker may be a non-functional amino acid sequence having a length of 1-20 or more amino acids and lacking a secondary or higher-order structure. For example, the linker is a flexible linker, such as a linker represented by $G_4S$, GS, GAP, $(G_4S)_2$, $(G_4S)_3$, $(G_4S)_4$, $(G_4S)_5$, or ASGS; in a further example, the linker is $(G_4S)_2$; for example, the linker is represented by $(G_mS_n)_h$ or $(G_mQ_n)_h$ or $(GGNGT)_h$ or $(YGNGT)_h$ or $(EPKSS)_h$, wherein m and n are each independently selected from the group consisting of integers of 1-8, and h is independently selected from the group consisting of integers of 1-20.

In some embodiments, the aforementioned IGF-1R/TSHR-binding protein comprises a sequence selected from the group consisting of the following:

[immunoglobulin single variable domain in first antigen-binding domain]-linker 1-Fc region-linker 2-[immunoglobulin single variable domain in second antigen-binding domain], and
[immunoglobulin single variable domain in second antigen-binding domain]-linker 1-Fc region-linker 2-[immunoglobulin single variable domain in first antigen-binding domain], wherein the linker 1 and the linker 2 may be independently present or absent, and the linker 1 and the linker 2 may be identical or different.

In some embodiments, the aforementioned IGF-1R/TSHR-binding protein comprises a combination of the following first and second polypeptide chains:

(1) a first polypeptide chain: [immunoglobulin single variable domain in first antigen-binding domain] 1-linker 1-Fc1-linker 2-[immunoglobulin single variable domain in first antigen-binding domain] 2, and
a second polypeptide chain: [immunoglobulin single variable domain in second antigen-binding domain] 1-linker 1-Fc2-linker 2-[immunoglobulin single variable domain in second antigen-binding domain] 2; or
(2) a first polypeptide chain: [immunoglobulin single variable domain in first antigen-binding domain] 1-linker 1-Fc2-linker 2-[immunoglobulin single variable domain in first antigen-binding domain] 2, and

a second polypeptide chain: [immunoglobulin single variable domain in second antigen-binding domain] 1-linker 1-Fc1-linker 2-[immunoglobulin single variable domain in second antigen-binding domain] 2,
wherein the linker 1 and the linker 2 may be independently present or absent, and the linker 1 and the linker 2 may be identical or different; the [immunoglobulin single variable domain in first antigen-binding domain] 1 and the [immunoglobulin single variable domain in first antigen-binding domain] 2 may be identical or different, and the [immunoglobulin single variable domain in second antigen-binding domain] 1 and the [immunoglobulin single variable domain in second antigen-binding domain] 2 may be identical or different.

In some specific embodiments, the Fc1 comprises one or more amino acid substitutions selected from the group consisting of 354C, 356E, 358M, and 366W, and the Fc2 comprises one or more amino acid substitutions selected from the group consisting of 349C, 356E, 358M, 366S, 368A, and 407V. For example, the Fc1 comprises 354C/366W mutations, and the Fc2 comprises 349C/366S/368A/407V mutations.

In some specific embodiments, the amino acid sequences of the linker 1 and the linker 2 are represented by $(G_mS_n)_h$ or $(G_mQ_n)_h$ or $(GGNGT)_h$ or $(YGNGT)_h$ or $(EPKSS)_h$, wherein m and n are each independently selected from the group consisting of integers of 1-8, and h is independently selected from the group consisting of integers of 1-20; in some specific embodiments, the linker 1 is absent, and the amino acid sequence of the linker 2 is represented by $(G_4Q)_3$ or $(G_4S)_3$.

In some embodiments, the aforementioned IGF-1R/TSHR-binding protein is an anti-IGF-1R/TSHR antibody or an antigen-binding fragment thereof. In some specific embodiments, the antibody is a bispecific antibody or a multispecific antibody. In some specific embodiments, the antibody is a camelid antibody, a chimeric antibody, a humanized antibody, or a fully human antibody. In some specific embodiments, the antibody or the antigen-binding fragment thereof is a recombinant antibody or a fragment thereof. In some specific embodiments, the antibody or the antigen-binding fragment thereof is a linear antibody, a single-chain antibody, a nanobody, a peptibody, a domain antibody, a diabody, a triabody, a tetrabody, a tandem di-scFv, or a tandem tri-scFv.

In some embodiments, the immunoglobulin single variable domain in the aforementioned IGF-1R/TSHR-binding protein is a single-domain antibody or VHH.

In some embodiments, provided is an IGF-1R/TSHR-binding protein, comprising the amino acid sequence set forth in any one of SEQ ID NOs: 49-52 or an amino acid sequence having at least 80% (preferably at least 90%) identity to any one of SEQ ID NOs: 49-52.

In some embodiments, the aforementioned IGF-1R/TSHR-binding protein has a function or property selected from the group consisting of at least one of the following:

(a) Binding to human TSHR or an epitope thereof with a $K_D$ value (M) of $\leq 10^{-7}$, and binding to human IGF-1R or an epitope thereof with a $K_D$ value (M) of $\leq 10^{-7}$. The method for determining the $K_D$ value is conventional in the art, for example, the method in Example 3 of the present disclosure.

(b) Inhibition of TSHR downstream signaling pathways, such as pAKT. In some embodiments, the $IC_{50}$ (nM) for inhibition of pAKT in A549 cells is less than 10, less than 5, less than 2, less than 1, less than 0.5, or less than 0.2. In some embodiments, the $IC_{50}$ value is determined by the method in Example 4 of the present disclosure.

(c) Inhibition of cAMP signals. In some embodiments, the $IC_{50}$ (nM) for inhibition of cAMP in HEK293 cells is less than 20, less than 10, less than 5, or less than 3. In some embodiments, the $IC_{50}$ value is determined by the method in Example 5 of the present disclosure.

(d) Inhibition of serum total thyroxine T4 and/or free T4. In some embodiments, T4 is detected according to the method in Example 6 of the present disclosure.

(e) No induction or almost no induction of an ADCC effect. In some embodiments, the ADCC is detected according to the method in Example 8 of the present disclosure.

(f) Synergistic inhibition of pERK by the first binding domain that specifically binds to TSHR and the second binding domain that specifically binds to IGF-1R. In some embodiments, pERK is detected according to the method in Example 9 of the present disclosure.

(g) Synergistic inhibition of hyaluronic acid secretion by cells (e.g., human orbital fibroblasts) by the first binding domain that specifically binds to TSHR and the second binding domain that specifically binds to IGF-1R. In some embodiments, the inhibition of the secretion of hyaluronic acid by cells is detected according to the method in Example 10 of the present disclosure.

In some embodiments, the aforementioned IGF-1R/TSHR-binding protein of the present disclosure may bind to TSHR with a $K_D$ value of $\leq 1 \times 10^{-8}$ M, e.g., $\leq 1 \times 10^{-9}$ M, or $\leq 1 \times 10^{-10}$ M, or $\leq 1 \times 10^{-11}$ M, or $\leq 1 \times 10^{-12}$ M. The aforementioned IGF-1R/TSHR-binding protein binds to IGF-1R with a $K_D$ value of $\leq 1 \times 10^{-8}$ M, or $\leq 1 \times 10^{-9}$ M, or $\leq 1 \times 10^{-10}$ M. In some embodiments, the aforementioned IGF-1R/TSHR-binding protein of the present disclosure encompasses a variant, wherein the variant comprises one or more (e.g., 1, 2, 3, 4, 5, 6, 7, 8, 9, or 10) amino acid mutations as compared to any one of SEQ ID NOs: 49-52; the amino acid mutations may be conservative replacements, substitutions, or modifications, and/or deletions or additions that do not affect function; the amino acid mutations may occur in the CDRs and/or FRs.

In some embodiments, provided is an IGF-1R/TSHR-binding protein (e.g., an anti-IGF-1R/TSHR antibody or an antigen-binding fragment thereof) that binds to or competes for binding to the same TSHR (e.g., human TSHR) and/or IGF-1R (e.g., human IGF-1R) epitope with the aforementioned IGF-1R/TSHR-binding protein of the present disclosure. In some embodiments, provided is an IGF-1R/TSHR-binding protein (e.g., an anti-IGF-1R/TSHR antibody or an antigen-binding fragment thereof) that blocks the binding of the aforementioned IGF-1R/TSHR-binding protein of the present disclosure to TSHR (e.g., human TSHR) and/or IGF-1R (e.g., human IGF-1R).

In some embodiments, provided is an IGF-1R/TSHR-binding protein (e.g., an anti-IGF-1R/TSHR antibody or an antigen-binding fragment thereof) whose binding to TSHR (e.g., human TSHR) and/or IGF-1R (e.g., human IGF-1R) is blocked by the immunoglobulin single variable domain in the aforementioned IGF-1R/TSHR-binding protein of the present disclosure.

In some embodiments, provided is a protein or molecule, comprising any one or more (e.g., 1, 2, 3, 4, 5, 6, 7, 8, 9, or 10) of the immunoglobulin single variable domains in the aforementioned TSHR-binding proteins of the present disclosure (the immunoglobulin single variable domains being identical or different) and comprising any one or more (e.g., 1, 2, 3, 4, 5, 6, 7, 8, 9, or 10) of the immunoglobulin single variable domains in the aforementioned IGF-1R-binding proteins of the present disclosure (the immunoglobulin single variable domains being identical or different). For example, the protein or molecule is a conjugate, and the conjugate may, for example, comprise any detectable label.

Polynucleotide and Vector

The present disclosure provides a polynucleotide encoding the IGF-1R/TSHR-binding protein, the IGF-1R-binding protein, or the TSHR-binding protein of the present disclosure. The nucleic acid of the present disclosure may be an RNA, DNA, or cDNA. According to some embodiments of the present disclosure, the nucleic acid of the present disclosure is a substantially isolated nucleic acid.

The nucleic acid of the present disclosure may also be in the form of a vector, may be present in a vector, and/or may be part of a vector. The vector is, for example, a plasmid, cosmid, YAC, or viral vector. The vector may especially be an expression vector, i.e., a vector that provides for *in vitro* and/or *in vivo* (i.e., in suitable host cells, host organisms, and/or expression systems) expression of the IGF-1R/TSHR-binding protein, the IGF-1R-binding protein, or the TSHR-binding protein. The expression vector generally comprises at least one of the nucleic acids of the present disclosure, which is operably linked to one or more suitable expression regulatory elements (e.g., promoters, enhancers, and terminators). The selection of the elements and sequences thereof for expression in a particular host is common knowledge for those skilled in the art. Regulatory elements and other elements useful or necessary for expression of the IGF-1R/TSHR-binding protein, the IGF-1R-binding protein, and the TSHR-binding protein of the present disclosure include, for example, promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information about the amino acid sequences of the polypeptides of the present disclosure, and/or may be isolated from a suitable natural source.

Host Cell

The present disclosure provides a recombinant host cell that expresses or is capable of expressing one or more of the IGF-1R/TSHR-binding proteins, the IGF-1R-binding proteins, and the TSHR-binding proteins of the present disclosure and/or comprises the polynucleotide or vector of the present disclosure. In some embodiments, the host cell is a bacterial cell, a fungal cell, or a mammalian cell.

Bacterial cells include, for example, cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, for example, cells of the species of *Trichoderma, Neurospora,* and *Aspergillus;* or cells of the species of *Saccharomyces* (e.g., *Saccharomyces cerevisiae), Schizosaccharomyces* (e.g., *Schizosaccharomyces pombe), Pichia* (e.g., *Pichia pastoris* and *Pichia methanolica),* and *Hansenula.*

Mammalian cells include, for example, HEK293 cells, CHO cells, BHK cells, HeLa cells, and COS cells.

However, amphibian cells, insect cells, plant cells, and any other cells used in the art for expressing heterologous proteins may also be used in the present disclosure.

The host cell of the present disclosure is unable to develop into a complete plant or animal individual.

Preparation Method

The present disclosure provides a method for preparing an IGF-1R/TSHR-binding protein, an IGF-1R-binding protein, or a TSHR-binding protein, comprising: expressing the target protein in the aforementioned host cell and isolating the target protein from the host cell. Optionally, the method may further comprise a purification step, for example, purifying using an A or G Sepharose FF column containing an adjusted buffer to wash off non-specifically bound components, then eluting bound antibodies using a pH gradient method, detecting using SDS-PAGE, and collecting. Optionally, filtration and concentration are performed by conventional methods. Soluble mixtures and multimers can also be removed by conventional methods, such as molecular sieves and ion exchange. The resulting products need to be immediately frozen, such as at -70 °C, or lyophilized. Methods for producing and purifying antibodies are well known in the prior art and can be found in, for example, Antibodies: A Laboratory Manual, Cold Spring Harbor Press (chapters 5-8 and 15).

The engineered antibodies or antigen-binding fragments of the present disclosure can be prepared and purified by conventional methods. For example, cDNA sequences encoding heavy and light chains can be cloned and recombined into an expression vector. CHO cells can be stably transfected with recombinant immunoglobulin expression vectors. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. The antibodies can be filtered and concentrated by conventional methods. Soluble mixtures and multimers can also be removed by conventional methods, such as molecular sieves and ion exchange.

Composition

The present disclosure provides a composition, comprising the aforementioned IGF-1R/TSHR-binding protein, IGF-1R-binding protein, and/or TSHR-binding protein of the present disclosure. For example, provided is a pharmaceutical composition, comprising an amount of the aforementioned IGF-1R/TSHR-binding protein, IGF-1R-binding protein, and/or TSHR-binding protein that is effective in treating, alleviating, or preventing a disease, and at least one pharmaceutically acceptable excipient, diluent, or carrier.

In some specific embodiments, a unit dose of the pharmaceutical composition may comprise 0.01 to 99 wt% of the IGF-1R/TSHR-binding protein, the IGF-1R-binding protein, and/or the TSHR-binding protein, or a unit dose of the pharmaceutical composition comprises 0.1-2000 mg of the IGF-1R/TSHR-binding protein, the IGF-1R-binding protein, and/or the TSHR-binding protein. In some specific embodiments, a unit dose of the pharmaceutical composition comprises 1-1000 mg of the IGF-1R/TSHR-binding protein, the IGF-1R-binding protein, and/or the TSHR-binding protein.

In some embodiments, provided is an article of manufacture or product (as an example, a kit), comprising the aforementioned IGF-1R/TSHR-binding protein, IGF-1R-binding protein, and/or TSHR-binding protein. Optionally, the article of manufacture comprises a container and a label. The container includes, for example, a bottle, a syringe, and a test tube. The container accommodates a composition effective in treating a disorder. The label on or associated with the container indicates that the composition is used for treating the disorder of choice.

In some embodiments, the aforementioned disease is an inflammatory disease or an autoimmune disease.

In some embodiments, the disease is Graves' orbitopathy.

Treatment Method and Pharmaceutical Use

The present disclosure provides a method for treating, alleviating, preventing, or diagnosing a disease or disorder by using the aforementioned IGF-1R/TSHR-binding protein, IGF-1R-binding protein, or TSHR-binding protein, coding polynucleotide therefor, or composition (including the pharmaceutical composition) thereof.

In some embodiments, provided is a method for ameliorating, alleviating, treating, or preventing a disease, comprising administering to a subject an amount of any one selected from the group consisting of the following that is effective in ameliorating, alleviating, treating, or preventing the disease:

1) the aforementioned IGF-1R/TSHR-binding protein or coding polynucleotide therefor, or pharmaceutical composition thereof of the present disclosure;
2) the aforementioned IGF-1R-binding protein or coding polynucleotide therefor, or pharmaceutical composition thereof of the present disclosure;
3) the aforementioned TSHR-binding protein or coding polynucleotide therefor, or pharmaceutical composition thereof of the present disclosure; and
4) the aforementioned combination of the IGF-1R-binding protein and the TSHR-binding protein, or coding polynucleotide therefor, or pharmaceutical composition thereof of the present disclosure.

In some embodiments, provided is use of the aforementioned items 1) to 4) in the manufacture of a medicament for ameliorating, alleviating, treating, or preventing a disease.

In some embodiments, the aforementioned disease is a disease or disorder caused by overexpression of IGF-1R and/or TSHR.

In some embodiments, the aforementioned disease is an autoimmune system disease.

In some embodiments, the aforementioned disease is an eye disease, such as Graves' orbitopathy (GO).

Detection

The present disclosure provides use of the IGF-1R/TSHR-binding protein, the IGF-1R-binding protein, the TSHR-binding protein, or the composition for detection. The present disclosure further provides a method, system, or device for detecting IGF-1R and/or TSHR *in vivo* or *in vitro,* comprising treating a sample with the aforementioned binding protein or composition of the present disclosure.

In some embodiments, the method, system, or device for *in vitro* detection may comprise, for example:

(1) contacting a sample with the IGF-1R/TSHR-binding protein, the IGF-1R-binding protein, the TSHR-binding protein, or the composition of the present disclosure;
(2) detecting a complex formed between the aforementioned binding protein and the sample; and/or
(3) contacting a reference sample (e.g., a control sample) with the binding protein; and
(4) determining the extent of formation of the complex by comparison with the reference sample. A change (e.g., a statistically significant change) in complex formation in the sample as compared to the control sample indicates the presence of IGF-1R and/or TSHR in the sample.

In some embodiments, further provided is a kit, comprising the aforementioned IGF-1R/TSHR-binding protein, IGF-1R-binding protein, or TSHR-binding protein, coding polynucleotide therefor, or composition thereof, wherein the kit may further comprise instructions for diagnostic use. The kit may further comprise at least one additional reagent, such as a label or an additional diagnostic agent. For *in vivo* use, the IGF-1R/TSHR-binding protein, the IGF-1R-binding protein, or the TSHR-binding protein, or the coding polynucleotide therefor may be formulated into a pharmaceutical composition.

## BRIEF DESCRIPTION OF THE DRAWINGS

FIG. 1A shows detection of the inhibition of IGF1-induced pAKT signals by the antibodies of the present disclosure and positive control antibodies in human non-small cell lung cancer cells (A549).

FIG. 1B shows detection of the inhibition of IGF1-induced pAKT signals by the antibodies of the present disclosure and positive control antibodies in mouse myoblasts (C2C12).

FIGs. 2A to 2C show results of cellular cAMP inhibition assays performed in HEK293 cells transiently transfected with human/rat/mouse TSHR.

FIGs. 3A to 3B show an efficacy assay of TSHR antibodies in Wistar rats, in which FIG. 3A shows results of total T4 concentration in serum, and FIG. 3B shows results of free T4 concentration in serum.

FIG. 4 shows results of *in vitro* ADCC effect detection for a bispecific antibody and positive control antibodies.

FIG. 5 shows results of synergistic inhibition of ERK phosphorylation in HEK293-TSHR cells by an IGF-1R/TSHR bispecific antibody.

FIG. 6 shows results of synergistic inhibition of hyaluronic acid production by primary orbital fibroblast cells by an IGF-1R/TSHR bispecific antibody.

## DETAILED DESCRIPTION

### Terminology

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure pertains. The three-letter and single-letter codes for amino acids used in the present disclosure are as described in J. biol. chem., 243, p3558 (1968).

"IGF-1R" (insulin-like growth factor-I receptor) belongs to the tyrosine protein kinase receptor family and is a widely expressed heterotetrameric protein. The heterotetrameric protein is involved in the regulation of proliferation and metabolic functions of many cell types and can be activated by IGF-1 and IGF-2 (both being insulin-like growth factors). IGF-1R is a tyrosine kinase receptor comprising two subunits. IGF-IR$\alpha$ comprises a ligand-binding domain, and IGF-IR$\beta$ is involved in signaling and comprises a tyrosine phosphorylation site. Illustratively, the sequence of human IGF-1R can be found in Uniprot: P08069.

"TSHR" (thyroid-stimulating hormone receptor) is a G protein-coupled receptor present on the membrane of thyroid follicular cells. Under normal physiological conditions, TSHR regulates the growth, differentiation, and function of thyroid follicular cells by binding to thyroid-stimulating hormone (TSH) and signaling through the adenylate cyclase (AC-cAMP) pathway and the phospholipase C-diacylglycerol and phosphoinositide pathways. Illustratively, the sequence of human TSHR can be found in Uniprot: P16473.

"IGF-1R-binding protein" encompasses any molecule capable of specifically binding to an IGF-1R protein or an epitope thereof, including but not limited to antibodies targeting IGF-1R as defined in the present disclosure, antigen-binding fragments thereof, or conjugates thereof.

"TSHR-binding protein" encompasses any molecule capable of specifically binding to a TSHR protein or an epitope thereof, including but not limited to antibodies targeting TSHR as defined in the present disclosure, antigen-binding fragments thereof, or conjugates thereof.

In some embodiments, the "IGF-1R-binding protein" may comprise at least one (e.g., 1, 2, 3, 4, 5, 6, or more) single-domain antibody that specifically binds to IGF-1R in the examples of the present disclosure. The "TSHR-binding protein" may comprise at least one (e.g., 1, 2, 3, 4, 5, 6, or more) single-domain antibody that specifically binds to TSHR in the examples of the present disclosure.

In some embodiments, the "IGF-1R-binding protein" of the present disclosure may comprise, in addition to the immunoglobulin single variable domain of IGF-1R, a linker and/or a moiety with an effector function, such as a half-life extending moiety (e.g., an immunoglobulin single variable domain that binds to serum albumin) and/or a fusion partner (e.g., serum albumin) and/or a conjugated polymer (e.g., PEG) and/or an Fc region. In some embodiments, the "TSHR-binding protein" may comprise, in addition to the immunoglobulin single variable domain of TSHR, a linker and/or a moiety with an effector function, such as a half-life extending moiety (e.g., an immunoglobulin single variable domain that binds to serum albumin) and/or a fusion partner (e.g., serum albumin) and/or a conjugated polymer (e.g., PEG) and/or an Fc region.

"IGF-1R/TSHR-binding protein" encompasses any protein capable of specifically binding to IGF-1R and TSHR or any molecule comprising the protein, including but not limited to antibodies, polypeptides, fusion proteins of antibodies and polypeptides, or conjugates thereof. In some embodiments, the "IGF-1R/TSHR-binding protein" encompasses the bispecific antibodies against IGF-1R and TSHR in the examples of the present disclosure.

"Antibody" encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), so long as they exhibit the desired antigen-binding activity. An antibody may refer to an immunoglobulin, which is of a four-peptide-chain structure formed by two heavy chains and two light chains linked by interchain disulfide bonds. The heavy chain constant regions of immunoglobulins differ in amino acid composition and arrangement; therefore, they also differ in antigenicity. Accordingly, immunoglobulins can be divided into five classes, or isotypes of immunoglobulins, namely IgM, IgD, IgG, IgA, and IgE, and their corresponding heavy chains are $\mu$ chains, $\delta$ chains, $\gamma$ chains, $\alpha$ chains, and $\varepsilon$ chains, respectively. Igs of the same class can be divided into different subclasses based on the amino acid composition of their hinge regions and the number and positions of heavy chain disulfide bonds; for example, IgGs can be divided into IgG1, IgG2, IgG3, and IgG4. Light chains are divided into $\kappa$ or $\lambda$ chains based on differences in their constant regions. Each of the five Ig classes may have a $\kappa$ chain or $\lambda$ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable region comprises 3 hypervariable regions (HVRs) and 4 framework regions (FRs) with relatively conservative sequences. The 3 hypervariable regions determine the specificity of the antibody and thus are also known as complementarity-determining regions (CDRs). Each light chain variable region (VL) or heavy chain variable region (VH) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

The antibodies of the present disclosure may be polyclonal, monoclonal, xenogeneic, allogeneic, syngeneic, or modified forms thereof, with the monoclonal antibodies being especially applicable in various examples. Generally, the antibodies of the present disclosure are recombinant antibodies. As used herein, "recombinant" generally refers to such products as a cell, a nucleic acid, a protein, or a vector, and indicates that the cell, the nucleic acid, the protein, or the vector has been modified by introduction of a heterologous nucleic acid or protein or alteration of a natural nucleic acid or protein, or that the cell is derived from a cell modified in this way. For example, recombinant cells express genes that are not found within the native (non-recombinant) cellular form or express native genes that are originally abnormally expressed, expressed at low levels, or not expressed at all.

For the determination or definition of CDRs, the deterministic depiction of CDRs and identification of residues comprising binding sites of an antibody can be accomplished by resolving the structure of the antibody and/or resolving the structure of the antibody-ligand complex. This can be achieved by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28: 214-8). The Chothia numbering scheme is similar to the Kabat numbering scheme, except that it takes into account the positions of certain structural loop regions (see, e.g., Chothia et al., 1986, J. Mol. Biol., 196: 901-17; Chothia et al., 1989, Nature, 342: 877-83). The AbM numbering scheme adopts a computer program integration suite for modeling antibody structures manufactured by Oxford Molecular Group (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3: 194-198). The contact definition is based on the analysis of the available complex crystal structures (see, e.g., MacCallum et al., 1996, J. Mol. Biol., 5: 732-45). In the conformational definition, the positions of the CDRs can be identified as residues that contribute enthalpy to the antigen binding (see, e.g., Makabe et al., 2008, Journal of Biological Chemistry, 283: 1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues does not significantly affect the antigen binding. As used herein, a CDR may refer to a CDR defined by any method known in the art, including combinations of methods. The correspondence between the various numbering schemes is well known to those skilled in the art.

"Domain" of a polypeptide or protein refers to a folded protein structure that is capable of maintaining its tertiary structure independently of the rest of the protein. In general, a domain is responsible for a particular functional property of a protein, and in many cases, may be added, deleted, or transferred to other proteins without loss of functions of the rest of the protein and/or the domain.

"Immunoglobulin domain" refers to a globular region of an antibody chain (e.g., a chain of a conventional antibody with a four-peptide-chain structure or of a heavy-chain antibody) or a polypeptide essentially consisting of such globular regions.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are spaced apart by three "complementarity-determining regions" or "CDRs" referred to in the art and hereinafter as "complementarity-determining region 1" or "CDR1", "complementarity-determining region 2" or "CDR2", and "complementarity-determining region 3" or "CDR3". Thus, the general structure or sequence of an immunoglobulin variable domain can be expressed as follows: FR1-CDR1-FR2-CDR2-FR3-CDR3-FR4. A immunoglobulin variable domain possesses specificity for an antigen by virtue of having an antigen-binding site.

"Antibody framework (FR)" refers to a portion of a variable domain, which serves as a scaffold for the antigen-binding loops (CDRs) of the variable domain.

"Immunoglobulin single variable domain" is used to refer to an immunoglobulin variable domain (which may be a heavy or light chain domain, including a VH, VHH, or VL domain) that generally can form a functional antigen-binding site without interacting with other domains (variable or constant domains) (e.g., without VH/VL interactions as are required between the VH and VL domains of conventional four-chain monoclonal antibodies). Examples of "immunoglobulin single variable domains" include nanobodies (including VHHs, humanized VHHs, and/or camelized VHs, e.g., camelized human VHs), IgNARs, domains, (single-domain) antibodies as VH domains or derived from VH domains (such as dAbs™), and (single-domain) antibodies as VL domains or derived from VL domains (such as dAbs™). Immunoglobulin single variable domains based on and/or derived from heavy chain variable domains (such as VH or VHH domains) are generally preferred. A specific example of an immunoglobulin single variable domain is a "VHH domain" (or simply "VHH") as defined below.

"VHH", also known as a heavy-chain single-domain antibody, VHH, $V_HH$ domain, VHH antibody fragment, VHH antibody, or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). "VHH" is used to distinguish the variable domain from the heavy chain variable domain (which is referred to in the present disclosure as a "VH domain" or VH) and the light chain variable domain (which is referred to in the present disclosure as a "VL domain" or VL) present in conventional antibodies with a four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and highly efficient antigen recognition unit formed by a single immunoglobulin domain. The terms "heavy-chain single-domain antibody", "VHH domain", "VHH", "$V_HH$ domain", "VHH antibody fragment", "VHH antibody",

"Nanobody®", and "Nanobody® domain" ("Nanobody" is a trademark of Ablynx N.V., Ghent, Belgium) are used interchangeably. VHHs include, but are not limited to, natural antibodies produced by camelids, antibodies produced by camelids and then humanized, or antibodies obtained by screening using phage display techniques. The total number of amino acid residues in a VHH is generally in the range of 110 to 120, typically between 112 and 115. However, it should be noted that shorter and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240(2000) 185-195; Li et al., J Biol Chem., 287(2012) 13713-13721; Deffar et al., African Journal of Biotechnology Vol. 8(12), pp. 2645-2652, 17 June, 2009; and WO94/04678.

As is well known in the art for VH domains and VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as a CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences, and in some specific embodiments, may contain the human framework region sequence of IGHV3. Methods for humanization include, for example, protein surface amino acid humanization (resurfacing) and universal framework grafting method for antibody humanization (CDR grafting to a universal framework), i.e., "grafting" CDRs onto other "scaffolds" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the VBase human species sequence database, as well as in Kabat, E. A. et al., 1991 Sequences of Proteins of Immunological Interest, 5th edition. The humanized antibody of the present disclosure also includes humanized antibodies that are further subjected to CDR affinity maturation by phage display. In addition, in order to avoid the decrease in activity caused by the decrease in immunogenicity, the framework sequence in the human antibody variable region can be subjected to minimum reverse mutation or back mutation to maintain activity.

The term "affinity-matured" antibody refers to an antibody that has one or more changes in one or more hypervariable regions (HVRs) that result in an improvement in the affinity of the antibody for the antigen, as compared to the parent antibody that does not have such changes. For example, an "affinity-matured" IGF-1R-binding protein or anti-IGF-1R antibody has one or more changes in one or more CDRs that result in an increase in the affinity for the antigen as compared to its parent antibody. Affinity-matured antibodies can be prepared, for example, by methods known in the art as described below: Marks et al., 1992, Biotechnology 10: 779-783 or Barbas et al., 1994, Proc. Nat. Acad. Sci, USA 91: 3809-3813; Shier et al., 1995, Gene 169: 147-155; Yelton et al., 1995, Immunol. 155: 1994-2004; Jackson et al., 1995, J. Immunol. 154(7): 3310-9; Hawkins et al., 1992, J. Mol. Biol. 226(3): 889896; KS Johnson and RE Hawkins, "Affinity maturation of antibodies using phage display", Oxford University Press 1996.

Typically, the IGF-1R/TSHR-binding protein, the IGF-1R-binding protein, and the TSHR-binding protein of the present disclosure may bind to the antigen or target protein to be bound (i.e., IGF-1R or TSHR) with a dissociation constant $(K_D)$ of preferably $10^{-7}$ to $10^{-10}$ mol/L (M), more preferably $10^{-8}$ to $10^{-10}$ mol/L, and even more preferably $10^{-9}$ to $10^{-10}$ or less, and/or with an association constant (KA) of at least $10^{-7}$ M, preferably at least $10^{-8}$ M, more preferably at least $10^{-9}$ M, and more preferably at least $10^{-10}$ M, as measured in a Biacore, KinExA, or Fortibio assay. Any $K_D$ value greater than $10^{-4}$ M is generally considered to indicate non-specific binding. Specific binding of an antigen-binding protein to an antigen or epitope can be measured in any suitable manner known, including, for example, surface plasmon resonance (SPR) assay, Scatchard assay, and/or competitive binding assay (e.g., radioimmunoassay (RIA), enzyme immunoassay (EIA), and sandwich competitive assay) described in the present disclosure.

"Binding affinity" or "affinity" is used herein as a measure of the strength of a noncovalent interaction between two molecules (e.g., an antibody or a portion thereof and an antigen). The binding affinity between two molecules can be quantified by determining the dissociation constant $(K_D)$. $K_D$ can be determined by measuring the kinetics of complex formation and dissociation by using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. $K_D$ is related to ka and kd by the equation $K_D$ = kd/ka. The value of the dissociation constant can be determined directly by well-known methods, and calculations can even be performed for complex mixtures by, for example, those methods described in Caceci et al (1984, Byte 9: 340-362). For example, $K_D$ can be determined by using a dual filtration nitrocellulose filter binding assay such as that disclosed by Wong & Lohman (1993, Proc. Natl. Acad. Sci. USA 90: 5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), for example, by using the Biacore™ system or KinExA. The binding affinities associated with different molecular interactions, e.g., the binding affinities of different antibodies for a given antigen, can be compared by comparing the $K_D$ values of antibody/antigen complexes. Similarly, the specificity of an interaction can be evaluated by determining and comparing the $K_D$ value for the interaction of interest (e.g., a specific interaction between an antibody and an antigen) with the $K_D$ value for an interaction not of interest (e.g., a control antibody known not to bind to IGF-1R or TSHR).

"Conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. In addition, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when amino acid residues in groups exhibiting similar properties as described above are replaced, it will not result in a particular change in properties.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The percent homology between two sequences is a function of the number of matching or homologous positions shared by the two sequences divided by the number of compared positions $\times$ 100%. For example, in the optimal alignment of sequences, if 6 out of 10 positions in two sequences are matched or homologous, the two sequences are 60% homologous. In general, when two sequences are aligned, a comparison is performed to obtain the maximum percent homology.

"Nucleic acid" or "polynucleotide" are used interchangeably in the present disclosure to refer to any DNA or RNA molecule, either single- or double-stranded, and, if single-stranded, the molecule of its complementary sequence, preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Host cell" includes individual cells or cell cultures, which may be or have been the recipient of a vector for incorporation of a polynucleotide insert. The host cell includes progeny of a single host cell, and the progeny may not necessarily be identical (in morphology or genomic DNA complement) to the original parent cell due to natural, accidental, or intentional mutations. The host cell includes cells transfected and/or transformed *in vivo* with the polynucleotide of the present disclosure. "Cell", "cell line", and "cell culture" are used interchangeably, and all such designations include their progeny. It should also be understood that all progeny may not be precisely identical in DNA content due to intentional or unintentional mutations. Mutant progeny that have the same function or biological activity as those selected in the initially transformed cells are included.

"Inhibition" and "blocking" are used interchangeably and encompass both partial and complete inhibition/blocking. "Inhibition of growth" (e.g., involving cells) is intended to include any measurable reduction in cell growth.

"Giving", "administering", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids, e.g., therapeutic, pharmacokinetic, diagnostic, research, and experimental methods. The treatment of cells comprises contacting a reagent with the cells and contacting the reagent with a fluid, wherein the fluid is in contact with the cells. "Giving", "administering", and "treating" also mean treating, e.g., a cell, by a reagent, diagnosis, a binding composition, or by another cell *in vitro* and *ex vivo*. When applied to humans, veterinary medicine, or research subjects, they refer to therapeutic treatment, preventive or prophylactic measures, and research and diagnostic applications.

"Treating" or "treatment" refers to administering a therapeutic agent, such as a therapeutic agent comprising any one of the binding proteins of the present disclosure or a pharmaceutical composition thereof, either internally or externally, to a subject who has had, is suspected of having, or is predisposed to having one or more autoimmune diseases or symptoms thereof on which the therapeutic agent is known to have a therapeutic effect. Typically, the therapeutic agent is administered in an amount effective in alleviating one or more disease symptoms in the subject or population being treated, whether by inducing regression of such symptoms or inhibiting the development of such symptoms into any clinically measurable degree. The amount of therapeutic agent effective in alleviating any specific disease symptom (also referred to as the "therapeutically effective amount") may vary depending on factors such as the disease state, age, and body weight of the subject, and the ability of the drug to produce a desired therapeutic effect in the subject. Whether a disease symptom has been palliated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating a disease symptom of interest in a certain subject, they shall alleviate the disease symptom of interest in a statistically significant number of subjects as determined by any statistical testing method known in the art, such as the Student's t-test, Chi-square test, U-test by Mann and Whitney, Kruskal-Wallis test (H-test), Jonckheere-Terpstra test, and Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects. The subject of the present disclosure may be an animal or human subject.

"Optional" or "optionally" means that the event or circumstance subsequently described may, but does not necessarily, occur. This description includes the instance where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to". In the context of the mutations contained in the Fc region of the present disclosure, "/" represents "and"; for example, "354C/366W" represents "354C and 366W", that is, the Fc comprises mutations 354C and 366W; the amino acid positions of the mutations in the Fc region of the present disclosure are numbered according to the EU numbering scheme. "Subject" or "patient" in the present disclosure means a mammal, particularly a primate, and especially a human.

## Examples

The present disclosure is further described below with reference to examples, but these examples are not intended to limit the scope of the present disclosure. In the examples of the present disclosure, experimental methods without specific conditions indicated were generally conducted under conventional conditions, such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

## Example 1. Screening and Preparation of Single-Domain Antibodies Specifically Binding to Human IGF-1R and Human TSHR

1. Antigens and proteins for detection

Amino acid sequences of related proteins for screening and detection were designed using human IGF-1R (Uniprot: P08069) and human TSHR (Uniprot: P16473) as templates.

> hIGF-1R-ECD-avi-his (amino acid sequence of human IGF-1R extracellular region)

EICGPGIDIRNDYQQLKRLENCTVIEGYLHILLISKAEDYRSYRFPKLTVITEYLLL
FRVAGLESLGDLFPNLTVIRGWKLFYNYALVIFEMTNLKDIGLYNLRNITRGAIRI
EKNADLCYLSTVDWSLILDAVSNNYIVGNKPPKECGDLCPGTMEEKPMCEKTTI
NNEYNYRCWTTNRCQKMCPSTCGKRACTENNECCHPECLGSCSAPDNDTACV
ACRHYYYAGVCVPACPPNTYRFEGWRCVDRDFCANILSAESSDSEGFVIHDGEC
MQECPSGFIRNGSQSMYCIPCEGPCPKVCEEEKKTKTIDSVTSAQMLQGCTIFKG
NLLINIRRGNNIASELENFMGLIEVVTGYVKIRHSHALVSLSFLKNLRLILGEEQL
EGNYSFYVLDNQNLQQLWDWDHRNLTIKAGKMYFAFNPKLCVSEIYRMEEVT
GTKGRQSKGDINTRNNGERASCESDVLHFTSTTTSKNRIIITWHRYRPPDYRDLI
SFTVYYKEAPFKNVTEYDGQDACGSNSWNMVDVDLPPNKDVEPGILLHGLKP
WTQYAVYVKAVTLTMVENDHIRGAKSEILYIRTNASVPSIPLDVLSASNSSSQLIV
KWNPPSLPNGNLSYYIVRWQRQPQDGYLRHNYCSKDKIPIRKYADGTIDIEEV
TENPKTEVCGGEKGPCCACPKTEAEKQAEKEEAEYRKVFENFLHNSIFVPRPER
KRRDVMQVANTTMSSRSRNTTAADTYNITDPEELETEYPFFESRVDNKERTVISN
LRPFTLYRIDIHSCNHEAEKLGCSASNFVFARTMPAEGADDIPGPVTWEPRPENSI
FLKWPEPENPNGLILMYEIKYGSQVEDQRECVSRQEYRKYGGAKLNRLNPGNY
TARIQATSLSGNGSWTDPVFFYVQAKTGYENFIH*GGGGSGGGGS*GLNDIFEAQK
IEWHE*GGGGSGGGGS*HHHHHHHH                                        SEQ ID NO: 1

(Note: The human IGF-1R extracellular region sequence is single underlined; the GS linker is in italics; the avi tag is double underlined; the his tag is underlined by a dashed line)
> hTSHR-ECD-avi-his (amino acid sequence of human TSHR extracellular region, 6 amino acid mutations)

GMGCSSPPCECHQEEDFRVTCKDIQRIPSLPPSTQTLKLIET*C*LRTIPSHAFSNLPN
ISRIYVSIDVTLQQLESHSFYNLSKVTHIEIRNT*P*NLTYIDPDALKELPLLKFLGIFN
TGLKMFP*P*LTKVYST*E*IFFILEITDNPYMTSIP*R*NAFQGLCNETLTLKLYNNGFTS
VQGYAFNGTKLDAVYLNKNKYLTVIDKDAFGGVYSGPSLLDVSQTSVTALPSK
GLEHLKEL*R*ARNTWTL*GGGGGSGGGGS*GLNDIFEAQKIEWHE*GGGGSGGGGS*

HHHHHHHH                                                          SEQ ID NO: 2

(Note: The human TSHR extracellular region is single underlined; the 6 amino acid mutation sites for optimizing protein production are in italics and bold; the GS linker is in italics; the avi tag is double underlined; the his tag is indicated by a dashed line)
> hTSHR-ECD-camel Fc-avi-his (amino acid sequence of human TSHR extracellular region, 6 amino acid mutations)

GMGCSSPPCECHQEEDFRVTCKDIQRIPSLPPSTQTLKLIET*C*LRTIPSHAFSNLPN
ISRIYVSIDVTLQQLESHSFYNLSKVTHIEIRNT*P*NLTYIDPDALKELPLLKFLGIFN
TGLKMFP*P*LTKVYST*E*IFFILEITDNPYMTSIP*R*NAFQGLCNETLTLKLYNNGFTS
VQGYAFNGTKLDAVYLNKNKYLTVIDKDAFGGVYSGPSLLDVSQTSVTALPSK
GLEHLKEL*R*ARNTWTLLEISEPQPQPGCTCPKCPAPELPGGPSVFVFPPKPKDVL
SISGRPEVTCVVVDVGKEDPEVNFNWYIDGVEVRTANTKPKEEQFNSTYRVVSV
LTIQHQDWLTGKEFKCKVNNKALPAPIERTISKAKGQTREPQVYTLAPHREELA
KDTVSVTCLVKGFYPPDINVEWQRNRQPESEGAYATTLPQLDNDGTYFLYSKLS
VGKNTWQRGETFTCVVMHEALHNHYTQKSITQSSGK*GGGGGSGGGGS*GLNDI
FEAQKIEWHE*GGGGSGGGGS*HHHHHHHH                                    SEQ ID NO: 3

(Note: The human TSHR extracellular region is single underlined; the amino acid mutation sites for optimizing protein production are in italics and bold; the camel Fc is indicated by a thick underline; the GS linker is in italics; the avi tag is double underlined; the his tag is indicated by a dashed line. SEQ ID NO: 3 is the human TSHR extracellular region fused to the N-terminus of the camel Fc region for immunization of camels.)

2. Screening of single-domain antibodies specifically binding to human IGF-1R and human TSHR

Each of human IGF-1R (SEQ ID NO: 1) and human TSHR-camel Fc (SEQ ID NO: 3) was used as an antigen protein to immunize one Bactrian camel. Prior to immunization, 5 mL of camel plasma was collected, and the serum was separated. After Freund's complete adjuvant was mixed with the antigen in a volume ratio of 1:1, the camel was subjected to subcutaneous multi-site immunization every two weeks. The dose of the first immunization was 200 μg of protein, and the dose of the following four immunizations was 100 μg of protein per immunization. A total of five immunizations were performed, and the titer was then determined. Plates (100 μL/well) were coated with 2 μg/mL human IGF-1R (SEQ ID NO: 1) and human TSHR (SEQ ID NO: 2) proteins, respectively, and incubated at 4 °C overnight. On the following day, the plates were washed and then blocked with 4% skim milk powder at 37 °C for 1 h. After washing, the camel serum was diluted in different gradients and then added, followed by incubation at 37 °C for 1 h. The negative controls were pre-immunization serum (diluted 1:1000) and a PBS solution. After the incubation was completed, the plates were washed three times with PBST, and an HRP-labeled rabbit anti-camel polyclonal antibody (diluted 1:1000) was added. The plates were then incubated at 37 °C for 1 h. After washing, an alkaline phosphatase chromogenic solution was added, the reaction was stopped with 2 M sulfuric acid, and the absorbance was measured at a wavelength of 450 nm. After a 1:1000000 dilution, the titer was detected. The titer was qualified, and peripheral blood from the camel was collected for library construction.
Lymphocytes were isolated from the camel peripheral blood, yielding a cell count of $1.2 \times 10^8$. RNA was extracted and reverse-transcribed to obtain cDNA, and a phage library was constructed. Single-domain antibodies with high affinity for human IGF-1R and human TSHR antigen proteins were obtained by screening of the phage library. 20 μg of biotinylated human IGF-1R (SEQ ID NO: 1) and human TSHR (SEQ ID NO: 2) were used to bind to 100 μL of Dynabeads™ M-280 Streptavidin. After standing at 37 °C for 1 h, the beads were blocked with 2% skim milk at room temperature for 1 h. The camel heavy-chain single-domain antibody phage display library was added, and the mixture was incubated at room temperature for 1 h. The beads were washed 9 times with PBST (PBS containing 0.05% Tween-20) to remove unbound phages. Phages specifically binding to human IGF-1R and human TSHR were eluted with 1 mg/mL trypsin and then used to infect *Escherichia coli* TG1 cells in the logarithmic growth phase. Then, phages were produced and purified for the next round of screening. A total of two rounds of screening were performed. From the enriched positive clones obtained after screening, 96 monoclonal colonies were picked. These monoclonal colonies were packaged into phage single-chain antibodies for phage ELISA testing. ELISA plates were coated with 2 μg/mL human IGF-1R and human TSHR antigens, respectively, and a phage supernatant diluted with the blocking solution was added. An assay was performed using an anti-M13 HRP-labeled antibody. Clones with OD450 value/background value >5 in the ELISA binding assay results were sequenced to obtain enriched single-domain antibody sequences.

For the target TSHR, cell-based phage library screening was also performed. HEK293 cells were transiently transfected with TSHR (37 °C, 5% $CO_2$). After 48 h, $2 \times 10^7$ cells were collected by centrifugation at 210 g for 6 min. The cells (HEK293-TSHR) and phage library were each blocked with PBS containing 5% BSA for 1 h at room temperature. The HEK293-TSHR transiently transfected cells were then incubated with the phage library at room temperature for 1 h. The cells were washed 5 times with PBST (PBS containing 0.05% Tween-20) and 3 times with PBS, followed by centrifugation at 210 g for 6 min. Bound phages were eluted with 0.5 mL of citric acid (0.1 M, pH 2.2) for 10 min and then neutralized with 0.5 mL of 1 M tris-HCl (pH 7.4). The eluted phages were used to infect *Escherichia coli* TG1 cells in the logarithmic growth phase, followed by phage production and purification for the next round of screening. A total of two rounds of screening were performed. After NGS sequencing, enriched sequences were selected for cell-based binding (flow cytometry) and functional verification.

After affinity determination, single-domain antibodies IG05 and IG06 targeting IGF-1R and a single-domain antibody CB16 targeting TSHR were selected.

> IG05

*QVQLVESGGGLVQAGGSLRLSCTASGLTFG*<u>TFGMA</u>*WFRQAPGKEREGVS*<u>AISASSG</u>

<u>RTYYADS</u>*VKGRFTISRDNAKNTLYLQMDALKPEDTAMYYCAV*<u>RSKNGAGYGFWY</u>

<u>EARLYGY</u>*WGQGTQVTVSS*                                   SEQ ID NO: 4

> IG06

*QVQLVESGGGSVQAGGSLRLSCAASRYIVS*<u>TRCLG</u>*WVRQAPGNEREEIA*<u>TIDTAGRP</u>

<u>TYADS</u>*VKGRFTISKDGAKNFLYLQMNSLKPEDTAMYYCAA*<u>ASSDNCRYFPSN</u>*LGQ*

*GTQVTVSS*                                             SEQ ID NO: 5

> CB 16

*EVQLVESGGGPVQAGGSLRLSCAGPGYTSS*<u>CMG</u>*WFRQAPGKERTGVA*<u>AIYIGGRGT</u>

<u>DYADS</u>*VKGRFTISQDNAKNTLYLQMNSLKPEDTAMYYCWA*<u>GIEYDCYSGHWRDW</u>

<u>AFSRERGF</u>*WGQGTQVTVSS*                                 SEQ ID NO: 6

In the above sequences SEQ ID NOs: 4-6, the order is FR1-CDR1-FR2-CDR2-FR3-CDR3-FR4. In the sequences, the FR sequences are in italics, and the underlined portions are the CDR1, CDR2, and CDR3 sequences, respectively. The CDR sequences are summarized in Table 1.

Table 1. Single-domain antibody CDRs (Kabat numbering scheme)

| Antibody No. | CDR sequences | | Sequence No. |
|---|---|---|---|
| IG05 | CDR1 | TFGMA | SEQ ID NO:7 |
| | CDR2 | AISASSGRTYYADSVKG | SEQ ID NO:8 |
| | CDR3 | RSKNGAGYGFWYEARLYGY | SEQ ID NO:9 |
| IG06 | CDR1 | TRCLG | SEQ ID NO:10 |
| | CDR2 | TIDTAGRPTYADSVKG | SEQ ID NO:11 |
| | CDR3 | ASSDNCRYFPSN | SEQ ID NO:12 |
| CB16 | CDR1 | CMG | SEQ ID NO:13 |
| | CDR2 | AIYIGGRGTDYADSVKG | SEQ ID NO:14 |
| | CDR3 | GIEYDCYSGHWRDWAFSRERGF | SEQ ID NO:15 |

3. Preparation of intact antibodies using single-domain antibodies

Intact antibodies were constructed using the aforementioned single-domain antibodies (VHHs). Specifically, the VHH sequences were each fused to the human IgG1-Fc region (CH2-CH3) sequence and constructed into a pTT5 expression vector. The sequence of the linked human IgG1-Fc may be as shown below:

> Human IgG1 Fc region

EPKSSDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPG

SEQ ID NO: 16

The following are the full sequences of IG05-IgG1, IG06-IgG1, and CB16-IgG1, which are IG05, IG06, and CB16 fused to the human IgG1 Fc region, respectively. Their sequences are shown below:

> IG05-IgG1

QVQLVESGGGLVQAGGSLRLSCTASGLTFG*TFGMA*WFRQAPGKEREGVS*AISAS*
*SGRTYYADSVKG*RFTISRDNAKNTLYLQMDALKPEDTAMYYCAV*RSKNGAGY*
*GFWYEARLYGY*WGQGTQVTVSS*EPKSSDKTHTCPPCPAPELLGGPSVFLFPPKP*
*KDTLMISRTPEVTCVVVDVSHEDPEVKFNWYVDGVEVHNAKTKPREEQYNSTYRVVS*
*VLTVLHQDWLNGKEYKCKVSNKALPAPIEKTISKAKGQPREPQVYTLPPSRDELTKN*
*QVSLTCLVKGFYPSDIAVEWESNGQPENNYKTTPPVLDSDGSFFLYSKLTVDKSRWQ*
*QGNVFSCSVMHEALHNHYTQKSLSLSPG*

SEQ ID NO: 17

> IG06-IgG1

QVQLVESGGGSVQAGGSLRLSCAASRYIVS<u>TRCLG</u>WVRQAPGNEREEIA<u>TIDTA GRPTYADS</u>VKGRFTISKDGAKNFLYLQMNSLKPEDTAMYYCAA<u>ASSDNCRYFP SN</u>LGQGTQVTVSS*EPKSSDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSVMH EALHNHYTQKSLSLSPG*

SEQ ID NO: 18

> CB16-IgG1

EVQLVESGGGPVQAGGSLRLSCAGPGYTSS<u>CMG</u>WFRQAPGKERTGVAAIYIGG RGTDYADSVKGRFTISQDNAKNTLYLQMNSLKPEDTAMYYCWAGIEYDCYSGH WRDWAFSRERGFWGQGTQVTVSS*EPKSSDKTHTCPPCPAPELLGGPSVFLFPPKP KDTLMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRDELTKN QVSLTCLVKGFYPSDIAVEWESNGQPENNYKTTPPVLDSDGSFFLYSKLTVDKSRWQ QGNVFSCSVMHEALHNHYTQKSLSLSPG*

SEQ ID NO: 19

The aforementioned antibody sequences were each cloned into a mammalian expression vector pTT5, and HEK293E or ExpiCHO cells were transfected with the vector for expression. The cell culture supernatant was harvested, filtered, and purified. Analysis showed that the protein of interest was obtained.

**Example 2. Engineering of Anti-IGF-1R and TSHR Single-Domain Antibodies**

1. Engineering by humanization

Three-dimensional structure homologous modeling was performed on the selected specific IGF-1R or TSHR single-domain antibodies. According to the modeling results, combined with the results of alignments with the V-base human germline sequence database and the IMGT human antibody heavy chain variable region germline gene database, the heavy chain variable region germline gene IGHV3-23 or IGHV3-66, which was highly homologous with the target sequence, was selected as a template for FR1, FR2, and FR3, and IGHJ4 was used as a template for FR4. The CDRs of the camelid-derived single-domain antibodies were grafted into the corresponding human templates, forming a variable region sequence in the order of FR1-CDR1-FR2-CDR2-FR3-CDR3-FR4. To keep the original activity of the single-domain antibodies after humanization, a series of back mutations were performed in the FRs.

Table 2. Humanization back mutation sites and amino acid types (Kabat numbering scheme)

| Antibody No. | The framework region (FR) comprises at least one amino acid mutation selected from the group consisting of the following |
|---|---|
| IG05 | 23T, 27L, 30G, 37F, 44E, 45R, 47G, 74A, 82D or A, and 94V |
| IG06 | 26R, 27Y, 28I, 44E, 45R, 47E or G, 48I, 49A, 73G, 74A, 77F, 94A, and 103L |
| CB16 | 24G, 25P, 27Y, 29S, 37F, 44E, 45R, 46T, 47G, 49A, 71Q, 74A, 93W, 94A |

The humanized sequences obtained are shown below, with the CDRs underlined (Kabat numbering scheme).

> IG05-hu1

EVQLVESGGGLVQPGGSLRLSCTASGLTFG<u>TFGMA</u>WFRQAPGKEREGVS<u>AISAS</u>
<u>SGRTYYADSVKG</u>RFTISRDNSKNTLYLQMNSLRAEDTAVYYCAVR<u>SKNGAGYG</u>
<u>FWYEARLYGY</u>WGQGTLVTVSS                                    SEQ ID NO: 20

> IG05-hu2

EVQLVESGGGLVQPGGSLRLSCTASGLTFG<u>TFGMA</u>WFRQAPGKEREGVS<u>AISAS</u>
<u>SGRTYYADSVKG</u>RFTISRDNAKNTLYLQMNSLRAEDTAVYYCAVR<u>SKNGAGYG</u>
<u>FWYEARLYGY</u>WGQGTLVTVSS                                    SEQ ID NO: 21

> IG05-hu3

EVQLVESGGGLVQPGGSLRLSCTASGLTFG<u>TFGMA</u>WFRQAPGKEREGVS<u>AISAS</u>
<u>SGRTYYADSVKG</u>RFTISRDNSKNTLYLQMDALRAEDTAVYYCAVR<u>SKNGAGYG</u>
<u>FWYEARLYGY</u>WGQGTLVTVSS                                    SEQ ID NO: 22

> IG05-hu4

EVQLVESGGGLVQPGGSLRLSCAASGLTFG<u>TFGMA</u>WFRQAPGKEREGVS<u>AISAS</u>
<u>SGRTYYADSVKG</u>RFTISRDNSKNTLYLQMNSLRAEDTAVYYCAVR<u>SKNGAGYG</u>
<u>FWYEARLYGY</u>WGQGTLVTVSS                                    SEQ ID NO: 23

> IG05-hu5

EVQLVESGGGLVQPGGSLRLSCAASGFTFG<u>TFGMA</u>WFRQAPGKEREGVS<u>AISAS</u>
<u>SGRTYYADSVKG</u>RFTISRDNSKNTLYLQMNSLRAEDTAVYYCAVR<u>SKNGAGYG</u>
<u>FWYEARLYGY</u>WGQGTLVTVSS                                    SEQ ID NO: 24

> IG06-hu1

EVQLVESGGGLVQPGGSLRLSCAASRYIVS<u>TRCLG</u>WVRQAPGKEREEVS<u>TIDTA</u>
<u>GRPTYADSVKG</u>RFTISRDNSKNTLYLQMNSLRAEDTAVYYCAA<u>ASSDNCRYFPS</u>
<u>N</u>WGQGTLVTVSS                                           SEQ ID NO: 25

> IG06-hu2

EVQLVESGGGLVQPGGSLRLSCAASRYIVS<u>TRCLG</u>WVRQAPGKEREEVSTIDTA
GRPTYADSVKGRFTISRDNSKNFLYLQMNSLRAEDTAVYYCAA<u>ASSDNCRYFPS</u>
<u>N</u>WGQGTLVTVSS                                           SEQ ID NO: 26

> IG06-hu3

EVQLVESGGGLVQPGGSLRLSCAASRYIVS<u>TRCLG</u>WVRQAPGKEREEVS<u>TIDTA</u>
<u>GRPTYADSVKG</u>RFTISRDGAKNFLYLQMNSLRAEDTAVYYCAA<u>ASSDNCRYFPS</u>
<u>N</u>WGQGTLVTVSS                                           SEQ ID NO: 27

> IG06-hu4

EVQLVESGGGLVQPGGSLRLSCAASRYIVSTRCLGWVRQAPGKEREEVSTIDTA
GRPTYADSVKGRFTISRDGAKNFLYLQMNSLRAEDTAVYYCAAASSDNCRYFPS
NLGQGTLVTVSS                                                    SEQ ID NO: 28

> IG06-hu5

EVQLVESGGGLVQPGGSLRLSCAASRYIVSTRCLGWVRQAPGKEREEIATIDTA
GRPTYADSVKGRFTISRDGAKNFLYLQMNSLRAEDTAVYYCAAASSDNCRYFPS
NLGQGTLVTVSS                                                    SEQ ID NO:
29

> IG06-hu6

EVQLVESGGGLVQPGGSLRLSCAASRYIVSTRCLGWVRQAPGNEREEVSTIDTA
GRPTYADSVKGRFTISRDNSKNTLYLQMNSLRAEDTAVYYCAAASSDNCRYFPS
NLGQGTLVTVSS                                                    SEQ ID NO: 30

> CB16-hu1

EVQLLESGGGLVQPGGSLRLSCAGPGYTSSCMGWFRQAPGKEREGVSAIYIGGR
GTDYADSVKGRFTISQDNSKNTLYLQMNSLRAEDTAVYYCWAGIEYDCYSGHW
RDWAFSRERGFWGQGTLVTVSS                                          SEQ ID NO: 31

> CB 16-hu2

EVQLLESGGGLVQPGGSLRLSCAGPGYTSSCMGWFRQAPGKERTGVAAIYIGGR
GTDYADSVKGRFTISQDNAKNTLYLQMNSLRAEDTAVYYCWAGIEYDCYSGH
WRDWAFSRERGFWGQGTLVTVSS                                         SEQ ID NO: 32

> CB16-hu3

EVQLLESGGGLVQPGGSLRLSCAGPGYTSSCMGWFRQAPGKERTGVSAIYIGGR
GTDYADSVKGRFTISQDNSKNTLYLQMNSLRAEDTAVYYCWAGIEYDCYSGHW
RDWAFSRERGFWGQGTLVTVSS                                          SEQ ID NO: 33

Based on IG05-hu5, TCEs and deamidation sites were engineered to obtain IG05-hu5-1.

> IG05-hu5-1

EVQLVESGGGLVQPGGSLRLSCAASGFTFGTFGMAWFRQAPGKEREGVSAISAS
SGRTYYADSVKGRFTISRDNSKNTLYLQMNSLRAEDTAVYYCAVRSKQGAGYG

FWYEGRLYGYWGQGTLVTVSS                                           SEQ ID NO: 34

That is, the CDRs of IG05 of the present disclosure have the following general structures:

CDR1: TFGMA (SEQ ID NO: 7)
CDR2: AISASSGRTYYADSVKG (SEQ ID NO: 8)

CDR3: RSKX$_1$GAGYGFWYEX$_2$RLYGY, wherein X$_1$ denotes N or Q, and X$_2$ denotes A or G (SEQ ID NO: 35).

The CDR3 may be RSKNGAGYGFWYEARLYGY (SEQ ID NO: 9) or RSKQGAGYGFWYEGRLYGY (SEQ ID NO: 36).

The method in Example 1 was used to construct full antibody sequences in which the C-terminus of the engineered single-domain antibody VHH was directly fused to the N-terminus of the hIgG1 Fc region (SEQ ID NO: 16). The antibody structures and their corresponding names are as follows:

Table 3. Antibody structures and corresponding names

| Antibody name | Sequence structure |
|---|---|
| IG05-IgG1 | The C-terminus of IG05 (SEQ ID NO: 4) is fused to the N-terminus of the IgG1 Fc region (SEQ ID NO: 16) |
| IG05-hu1-IgG1 | The C-terminus of IG05-hul (SEQ ID NO: 20) is fused to the N-terminus of the IgG1 Fc region (SEQ ID NO: 16) |
| IG05-hu2-IgG1 | The C-terminus of IG05-hu2 (SEQ ID NO: 21) is fused to the N-terminus of the IgG1 Fc region (SEQ ID NO: 16) |
| IG05-hu3-IgG1 | The C-terminus of IG05-hu3 (SEQ ID NO: 22) is fused to the N-terminus of the IgG1 Fc region (SEQ ID NO: 16) |
| IG05-hu4-IgG1 | The C-terminus of IG05-hu4 (SEQ ID NO: 23) is fused to the N-terminus of the IgG1 Fc region (SEQ ID NO: 16) |
| IG05-hu5-IgG 1 | The C-terminus of IG05-hu5 (SEQ ID NO: 24) is fused to the N-terminus of the IgG1 Fc region (SEQ ID NO: 16) |
| IG05-hu5-1-IgG1 | The C-terminus of IG05-hu5-1 (SEQ ID NO: 34) is fused to the N-terminus of the IgG1 Fc region (SEQ ID NO: 16) |
| IG06-IgG1 | The C-terminus of IG06 (SEQ ID NO: 5) is fused to the N-terminus of the IgG1 Fc region (SEQ ID NO: 16) |
| IG06-hu1-IgG1 | The C-terminus of IG06-hu2 (SEQ ID NO: 25) is fused to the N-terminus of the IgG1 Fc region (SEQ ID NO: 16) |
| IG06-hu2-IgG1 | The C-terminus of IG06-hu3 (SEQ ID NO: 26) is fused to the N-terminus of the IgG1 Fc region (SEQ ID NO: 16) |
| IG06-hu3-IgG1 | The C-terminus of IG06-hu4 (SEQ ID NO: 27) is fused to the N-terminus of the IgG1 Fc region (SEQ ID NO: 16) |
| IG06-hu4-IgG1 | The C-terminus of IG06-hu5 (SEQ ID NO: 28) is fused to the N-terminus of the IgG1 Fc region (SEQ ID NO: 16) |
| IG06-hu5-IgG1 | The C-terminus of IG06-hu6 (SEQ ID NO: 29) is fused to the N-terminus of the IgG1 Fc region (SEQ ID NO: 16) |
| IG06-hu6-IgG1 | The C-terminus of IG06-hu7 (SEQ ID NO: 30) is fused to the N-terminus of the IgG1 Fc region (SEQ ID NO: 16) |
| CB16-IgG1 | The C-terminus of CB16 (SEQ ID NO: 6) is fused to the N-terminus of the IgG1 Fc region (SEQ ID NO: 16) |
| CB16-hu1-IgG1 | The C-terminus of CB16-hu1 (SEQ ID NO: 31) is fused to the N-terminus of the IgG1 Fc region (SEQ ID NO: 16) |
| CB16-hu2-IgG1 | The C-terminus of CB16-hu2 (SEQ ID NO: 32) is fused to the N-terminus of the IgG1 Fc region (SEQ ID NO: 16) |
| CB16-hu3-IgG1 | The C-terminus of CB16-hu3 (SEQ ID NO: 33) is fused to the N-terminus of the IgG1 Fc region (SEQ ID NO: 16) |

Exemplary intact antibody sequences are as follows:

> IG05-hu5-1-IgG1

EVQLVESGGGLVQPGGSLRLSCAASGFTFG*TFGMA*WFRQAPGKEREGVS*AISAS SGRTYYADSVKG*RFTISRDNSKNTLYLQMNSLRAEDTAVYYCAV*RSKQGAGYG FWYEGRLYGY*WGQGTLVTVSS*EPKSSDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSRWQQG NVFSCSVMHEALHNHYTQKSLSLSPG*

<div align="right">SEQ ID NO: 37</div>

> CB16-hu3-IgG1

EVQLLESGGGLVQPGGSLRLSCAGPGYTSS*CMG*WFRQAPGKERTGVS*AIYIGGR GTDYADSVKG*RFTISQDNSKNTLYLQMNSLRAEDTAVYYCWA*GIEYDCYSGHW RDWAFSRERG*FWGQGTLVTVSS*EPKSSDKTHTCPPCPAPELLGGPSVFLFPPKPK DTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKSRWQQ GNVFSCSVMHEALHNHYTQKSLSLSPG*

<div align="right">SEQ ID NO: 38</div>

**Example 3. Determination of Affinity of Anti-IGF-1R Antibodies and Anti-TSHR Antibodies for Antigens**

1. Determination of affinity of anti-IGF-1R antibodies for human and murine IGF-1R The affinity of IG05- and IG06-related antibodies for IGF-1R was determined by a Biacore T200 (GE Healthcare) instrument, and the positive controls were Tepezza, VRDN-001, and PHP1003.

A Series S sensor chip CM5 (GE Healthcare, Cat. 29149603) was adopted, and an anti-His antibody kit (GE Healthcare, Cat. 28-9950-56) was immobilized onto the surface of the chip by amine coupling using an amine coupling kit (GE Healthcare, Cat. BR100050). Then, the human IGF-1R (SEQ ID NO: 1)/mouse IGF-1R (Acro, Cat. IGR-M5223) antigens were captured. The IG05- and IG06-related antibodies and positive control antibodies at different concentrations were allowed to flow over the surface of the chip. Reaction signals were detected in real time to obtain association and dissociation curves, and the binding capacity constant was obtained by fitting. The solution used in the experiment was an HBS-EP solution. At the end of each experimental cycle, the chip was regenerated with a pH 1.5 glycine (GE Healthcare, Cat. BR-1003-54) solution. The affinity results of the antibodies are shown in Table 4 and Table 5.

The results showed that the affinity of the IG05- and IG06-related intact antibodies obtained by screening of the present disclosure for human IGF-1R was superior to that of the positive controls Tepezza, VRDN-001, and PHP1003. In addition, IG06 exhibited good species cross-reactivity with mouse IGF-1R, and could be used as a surrogate antibody for *in vivo* efficacy studies in non-transgenic mice and rats.

Table 4. Affinity of IG05- and IG06-related single-domain antibodies for human/mouse IGF-1R

| Antibody name | Antigen | $k_a$ (1/Ms) | $k_d$ (1/s) | $K_D$ (M) |
|---|---|---|---|---|
| IG05 | | 2.17E+06 | 7.49E-04 | 3.46e-10 |
| IG05-hu1 | | 1.82E+06 | 7.20E-04 | 3.95e-10 |
| IG05-hu2 | | 2.14E+06 | 7.72E-04 | 3.61e-10 |
| IG05-hu3 | | 1.79E+06 | 7.23E-04 | 4.03e-10 |
| IG05-hu4 | | 1.74E+06 | 8.82E-04 | 5.07e-10 |

(continued)

| Antibody name | Antigen | $k_a$ (1/Ms) | $k_d$ (1/s) | $K_D$ (M) |
|---|---|---|---|---|
| IG05-hu5 | Human IGF-1R | 1.83E+06 | 4.74E-04 | 2.58e-10 |
| IG06 | | 7.59E+05 | 6.75E-04 | 8.89e-10 |
| IG06-hu1 | | 9.05E+05 | 2.65E-02 | 2.93e-08 |
| IG06-hu2 | | 8.00E+05 | 2.77E-02 | 3.47e-08 |
| IG06-hu3 | | 8.47E+05 | 2.31E-02 | 2.73e-08 |
| IG06-hu4 | | 7.48E+05 | 2.99E-03 | 4.00E-09 |
| IG06-hu5 | | 8.99E+05 | 2.40E-03 | 2.67E-09 |
| IG06-hu6 | | 1.46E+06 | 2.26E-03 | 1.55E-09 |

Table 5. Affinity of IG05- and IG06-related intact antibodies for human/mouse IGF-1R

| Antibody name | Antigen | $k_a$ (1/Ms) | $k_d$ (1/s) | $K_D$ (M) |
|---|---|---|---|---|
| IG05-hu5-1-IgG1 | Human IGF-1R | 8.87E+06 | 1.87E-05 | 2.11E-12 |
| IG06-IgG1 | | 5.07E+06 | 1.55E-04 | 3.06E-11 |
| Tepezza | | 4.08E+06 | 1.09E-03 | 2.67E-10 |
| VRDN-001 | | 8.39E+05 | 1.47E-05 | 1.75E-11 |
| PHP1003 | | 4.10E+06 | 1.07E-03 | 2.62E-10 |
| IG05-hu5-1-IgG1 | Mouse IGF-1R | / | / | / |
| IG06-IgG1 | | 5.27E+06 | 4.96E-04 | 9.43E-11 |
| Tepezza | | / | / | / |
| VRDN-001 | | / | / | / |
| PHP1003 | | 3.32E+06 | 1.12E-03 | 3.37E-10 |

2. Determination of affinity of anti-TSHR antibodies for human TSHR

The affinity of CB16-related antibodies for human TSHR was determined by a Biacore T200 (GE Healthcare) instrument, the positive controls were K1-70 and M22, and the negative control was HBS-EP (10 mM HEPES, 150 mM NaCl, 3 mM EDTA, 0.005% P20, pH 7.4).

The antibodies to be tested were captured to the surface of a chip (Series S sensor chip Protein A, GE Healthcare, Cat. 29127556), and human TSHR (SEQ ID NO: 2) at different concentrations was then allowed to flow over the surface of the chip. Reaction signals were detected in real time to obtain association and dissociation curves, and the binding capacity constant was obtained by fitting. The solution used in the experiment was an HBS-EP solution. At the end of each experimental cycle, the chip was regenerated with a pH 1.5 glycine (GE Healthcare, Cat. BR-1003-54) solution.

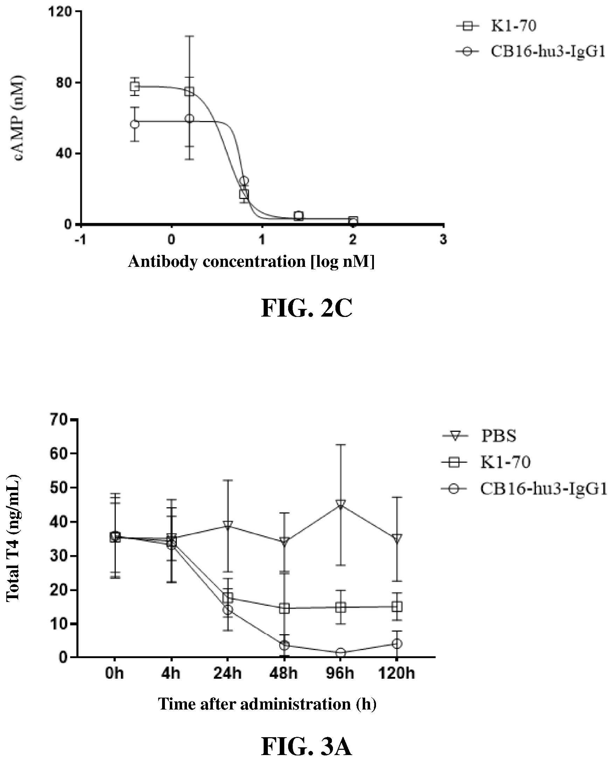

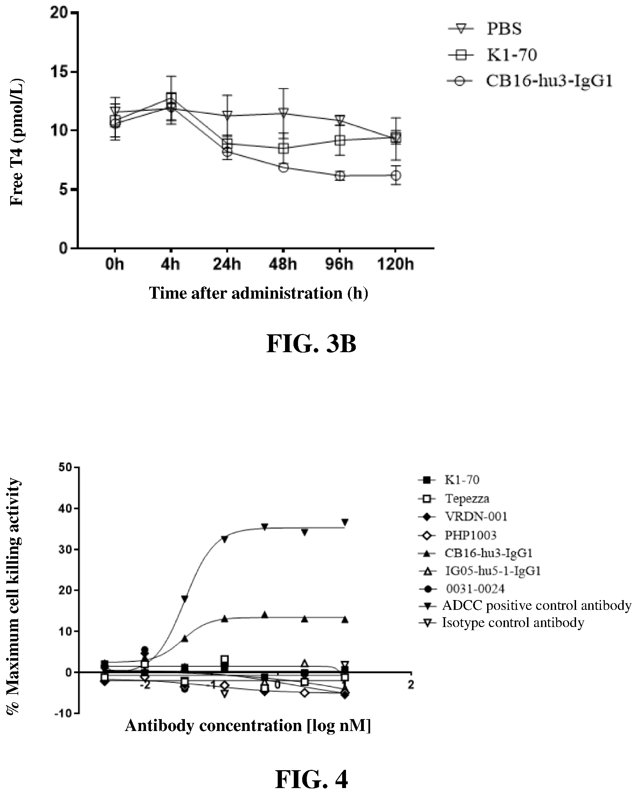

The affinity results of the antibodies are shown in Table 6. A subsequent *in vivo* efficacy study demonstrated that CB16-hu3-IgG1 was superior to K1-70 (FIGs. 3A and 3B).

Table 6. Affinity of anti-TSHR antibodies for human TSHR

| Antibody name | Antigen | $k_a$ (1/Ms) | $k_d$ (1/s) | $K_D$ (M) |
|---|---|---|---|---|
| CB16-IgG1 | Human TSHR | 1.88E+05 | 1.07E-03 | 5.67e-09 |
| CB16-hu1-IgG1 | | 1.43E+05 | 6.57E-03 | 4.60e-08 |
| CB16-hu2-IgG1 | | 1.78E+05 | 9.88E-04 | 5.54e-09 |
| CB16-hu3-IgG1 | | 1.67E+05 | 1.36E-03 | 8.14e-09 |
| K1-70 | | 5.30E+05 | 4.33E-04 | 8.15E-10 |
| M22 | | 1.05E+06 | 4.62E-03 | 4.41E-09 |

In the present disclosure, the positive controls of the anti-IGF-1R antibodies were Tepezza (Horizon, US2022356257), VRDN-001 (Viridian, WO2023019171), and PHP1003 (Suzhou PRO-HEAL, CN113512116), and the positive controls of the anti-TSHR antibodies were K1-70 (PDB: 7XW7, Av7) and the agonist antibody M22 (PDB: 3G04). Their sequences are shown below:

> Tepezza antibody heavy chain

QVELVESGGGVVQPGRSQRLSCAASGFTFSSYGMHWVRQAPGKGLEWVAIIWF
DGSSTYYADSVRGRFTISRDNSKNTLYLQMNSLRAEDTAVYFCARELGRRYFDL
WGRGTLVSVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPG                                          SEQ ID NO: 39

> Tepezza antibody light chain

EIVLTQSPATLSLSPGERATLSCRASQSVSSYLAWYQQKPGQAPRLLIYDASKR
ATGIPARFSGSGSGTDFTLTISSLEPEDFAVYYCQQRSKWPPWTFGQGTKVESK
RTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRG
EC                                          SEQ ID NO: 40

> VRDN-001 antibody heavy chain

QVQLVQSGAEVVKPGASVKLSCKASGYTFTSYWMHWVKQRPGQGLEWIGEIN
PSNGRTNYNQKFQGKATLTVDKSSSTAYMQLSSLTSEDSAVYYFARGRPDYYGS
SKWYFDVWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVF
SCSVMHEALHNHYTQKSLSLSPG                                          SEQ ID NO: 41

> VRDN-001 antibody light chain

DVVMTQTPLSLPVSLGDPASISCRSSQSIVHSNVNTYLEWYLQKPGQSPRLLIYK
VSNRFSGVPDRFSGSGAGTDFTLRISRVEAEDLGIYYCFQGSHVPPTFGGGTKLE
IKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE
C                                          SEQ ID NO: 42

> PHP1003 antibody heavy chain

QVQLQESGPGLVKPSGTLSLTCAVSGGSISSSNWWSWVRQPPGKGLEWIGEIYH
SGSTNYNPSLKSRVTISVDKSKNQFSLKLSSVTAADTAVYYCARWTGRTDAFDI
WGQGTMVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPEAAGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPG                                      SEQ ID NO: 43

> PHP1003 antibody light chain

DVVMTQSPLSLPVTPGEPASISCRSSQSLLHSNGYNYLDWYLQKPGQSPQLLIYL
GSNRASGVPDRFSGSGSGTDFTLKISRVEAEDVGVYYCMQGTHWPLTFGQGTK
VEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC                                                     SEQ ID NO: 44

> K1-70 antibody heavy chain

EVQLVQSGAEVKKPGQSLKISCKASGYSLTDNWIGWVRQKPGKGLEWMGIIYP
GDSDTRYSPSFQGQVTISADKSINTAYLQWSSLKASDTAIYYCVGLDWNYNPLR
YWGPGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM

HEALHNHYTQKSLSLSPG                                      SEQ ID NO: 45

> K1-70 antibody light chain

QSVLTQPPSVSAAPGQKVTISCSGSSSDIGSNYVSWYQQFPGTAPKLLIYDNNK
RPSAIPDRFSGSKSGTSATLGITGLQTGDEADYYCGTWDSRLGIAVFGGGTQLT
VLGQPKAAPSVTLFPPSSEELQANKATLVCLVSDFYPGAVTVAWKADGSPVKV
GVETTKPSKQSNNKYAASSYLSLTPEQWKSHRSYSCRVTHEGSTVEKTVAPTE
CS                                                      SEQ ID NO: 46

> M22 antibody heavy chain

QVQLVQSGAEVKKPGESLKISCRGSGYRFTSYWINWVRQLPGKGLEWMGRIDP
TDSYTNYSPSFKGHVTVSADKSINTAYLQWSSLKASDTGMYYCARLEPGYSST
WSVNWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPG                                       SEQ ID NO: 47

> M22 antibody light chain

LTVLTQPPSVSGAPRQRVTISCSGNSSNIGNNAVNWYQQLPGKAPKLLIYYDD
QLPSGVSDRFSGSRSGTSASLAIRGLQSEDEADYYCTSWDDSLDSQLFGGGTR
LTVLGQPKAAPSVTLFPPSSEELQANKATLVCLISDFYPGAVTVAWKADSSPVK
AGVETTTPSKQSNNKYAASSYLSLTPEQWKSHKSYSCQVTHEGSTVEKTVAP
TECS                                                     SEQ ID NO: 48.

**Example 4. Assay of Inhibition of Phosphorylated AKT (pAKT) by Anti-IGF-1R Antibodies**

To detect the effects of anti-IGF-1R antibodies on the human IGF-1R downstream signaling pathway, inhibition of IGF1-induced pAKT signals by the antibodies was examined.

pAKT levels were detected using PHOSPHO-AKT (SER473) KITS (PerkinElmer, Cat. No. 64AKSPET).

Human non-small cell lung cancer cells (A549, 15000 cells/well) and mouse myoblasts (C2C12, 15000 cells/well) were seeded in 96-well flat-bottom plates and cultured overnight in DMEM (Gibco, Cat. No. 11995065) supplemented with 1% FBS. After the start of the assay, the culture medium in the well plates was discarded and replaced with 90 $\mu$L of HANKS balanced salt solution. Each antibody to be tested was diluted, and 10 $\mu$L of the diluted antibody was added to the cells in each well. The mixture was mixed well. The well plates were incubated in a cell incubator at 37 °C for 30 min. After the incubation was completed, 10 $\mu$L of human IGF1 (Beyotime, Cat. No. P5502) was added to each well to a final concentration of 100 ng/mL, and incubation was performed at 37 °C for another 30 min.

After the incubation was completed, all liquid in the well plates was discarded. According to the instructions of PHOSPHO-AKT (SER473) KITS, 1× supplemented lysis buffer was added to lyse the cells, followed by shaking at 400 rpm at room temperature for 20 min. After that, 16 $\mu$L of the cell lysate was transferred to a white 96-well plate, and 4 $\mu$L of a mixture of Phospho-AKT d2 and Phospho-AKT Eu Cryptate antibodies was added. The plate was covered with a plate-sealing film and allowed to stand at room temperature for 4 h. Readings were taken at 665 nm and 620 nm using a Tecan Infinite® 200 PRO microplate reader. The values were calculated according to the following formula, and the ability of the antibodies to inhibit pAKT signals was compared.

$$Ratio = \frac{Signal665nm}{Signal620nm} \times 10000$$

IG05-hu5-1-IgG1, IG06-IgG1, PHP1003, VRDN-001, and Tepezza were all compared in A549 cells using the above method. Since IG06-IgG1 has species cross-reactivity with mice, the ability of IG06-IgG1 to bind to mouse IGF-1R to inhibit pAKT in C2C12 mouse myoblasts was also verified.

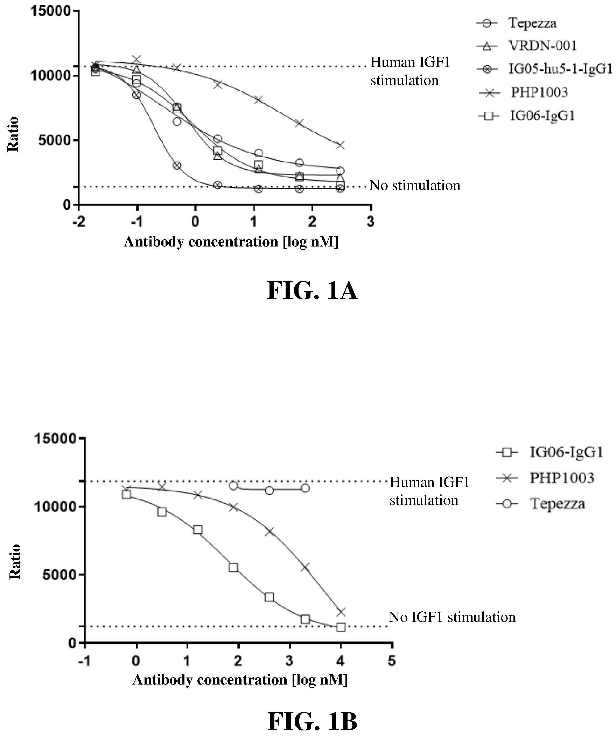

As shown in FIG. 1A and Table 7-1, in human non-small cell lung cancer cells (A549), Tepezza, VRDN-001, PHP1003, IG05-hu5-1-IgG1, and IG06-IgG1 could all inhibit IGF1-induced pAKT signals, with IG05-hu5-1-IgG1 exhibiting the strongest inhibitory effect. As shown in FIG. 1B and Table 7-2, in mouse myoblasts (C2C12), IG06-IgG1 and PHP1003 could inhibit IGF1-induced pAKT signals, wherein IG06-IgG1 exhibited a superior inhibitory function compared to PHP1003, while Tepezza exhibited no inhibitory effect due to a lack of species cross-reactivity with mice.

Table 7-1. Experimental results of inhibition of IGF1-induced pAKT signaling in A549 cells by antibodies

| Experimental group | IC$_{50}$ (nM) |
|---|---|
| Tepezza | NA |
| IG05-hu5-1-IgG1 | 0.1935 |
| VRDN001 | 0.7155 |
| PHP1003 | 33.6 |
| IG06-IgG1 | 0.8979 |

Table 7-2. Experimental results of inhibition of IGF1-induced pAKT signaling in C2C12 cells by antibodies

| Experimental group | IC$_{50}$ (nM) |
|---|---|
| IG06-IgG1 | 62.23 |
| PHP1003 | 4240 |
| Tepezza | / |
| Note: / indicates no pAKT inhibitory effect, NA indicates that no accurate IC$_{50}$ value was obtained. | |

## Example 5. Detection of Inhibitory Effects of Anti-TSHR Antibodies on cAMP Signaling

1. cAMP inhibition assay in HEK293 cells overexpressing human TSHR

To detect the effects of anti-TSHR antibodies on the human TSHR downstream signaling pathway, inhibition of TSH-induced cAMP signals by the antibodies was examined.

HEK293 suspension cells were transiently transfected with a plasmid expressing full-length human TSHR using lipofectamine 2000 (ThermoFisher, Cat. No. 11668019). After 48 h of culture, the cells were resuspended in HANKS balanced salt solution and seeded into 96-well U-bottom plates at 50000 cells/well, and pre-incubated with antibodies at different concentrations for 1 h. 5000 cells from each well were transferred to an HTRF 96-well plate (cisbio, Cat. No. 66PL96025), and bovine TSH (Sigma, Cat. No. T8931) at a final concentration of 0.1 μM or 100 ng/mL M22 was added for stimulation for 30 min. The concentration of cAMP produced by the lysed cells was detected using a CAMP-GS DYNAMIC KIT (cisbio, Cat. No. 62AM4PEB).

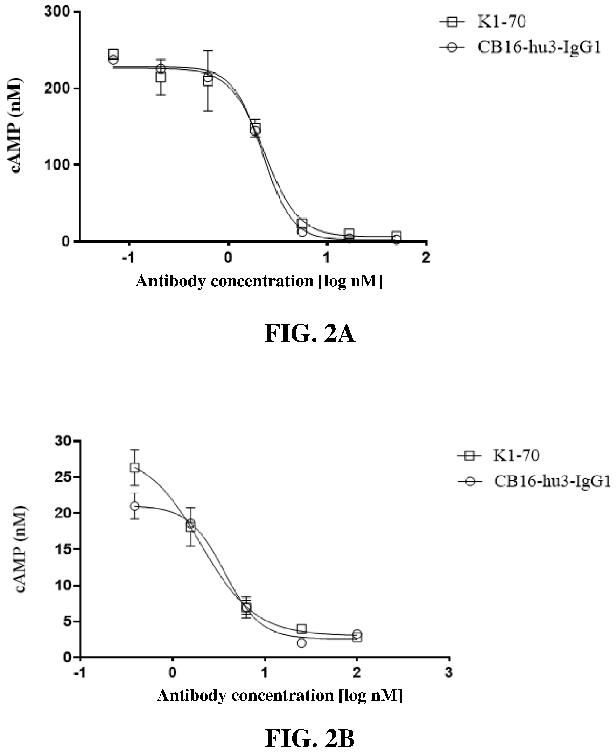

As shown in FIG. 2A, both CB16-hu3-IgG1 and the positive control antibody K1-70 exhibited inhibitory function against cAMP in HEK293-human TSHR cells, with IC$_{50}$ values of 2.202 nM and 2.295 nM, respectively, indicating comparable functional activity. These results demonstrated that CB16-hu3-IgG1 and K1-70 were capable of blocking the interaction between the ligand and the TSHR receptor and inhibiting the activation of its downstream cAMP signaling pathway.

2. cAMP inhibition assay in HEK293 cells overexpressing rat/mouse TSHR

This assay was intended to detect the inhibitory effects of anti-TSHR antibodies on downstream cAMP signals mediated by rat/mouse TSHR receptors.

HEK293 suspension cells were transiently transfected with a plasmid expressing full-length rat or mouse TSHR. After 48 h of culture, the cells were resuspended in HANKS balanced salt solution and seeded into 96-well U-bottom plates at 100000 cells/well, and pre-incubated with antibodies at different concentrations for 1 h. 10000 cells from each well were transferred to an HTRF 96-well plate, and HEK293-rat TSHR cells were stimulated with bovine TSH at a final concentration of 0.1 μM or HEK293-mouse TSHR cells were stimulated with 0.1 μM bovine TSH for 30 min. The concentration of cAMP produced by the lysed cells was detected using the CAMP-GS DYNAMIC KIT.

As shown in FIGs. 2B and 2C, both CB16-hu3-IgG1 and K1-70 were capable of significantly inhibiting bovine TSH-stimulated cAMP production mediated by rat and mouse TSHR, and their activities were similar. The inhibitory activity IC$_{50}$ values of CB16-hu3-IgG1 were 3.661 nM (rat) and 5.941 nM (mouse), respectively, and the inhibitory activity IC$_{50}$ values of K1-70 were 2.018 nM (rat) and 4.031 nM (mouse), respectively.

## Example 6. Efficacy Study of Anti-TSHR Antibodies in Wistar Rats

To evaluate the inhibitory function of TSHR antibodies *in vivo,* a Wistar rat model was selected to investigate the effects of the antibodies on total thyroxine (T4) and free T4 levels in rat serum under physiological conditions.

Five-week-old female Han Wistar rats (purchased from Beijing Vital River Laboratory Animal Technology Co., Ltd.) were divided into 3 groups based on body weight, with 5 rats per group. On Day 0, by intramuscular injection, each rat was given an equimolar amount of K1-70 positive control antibody (200 μg) or CB16-hu3-IgG1 (106.7 μg), while the control group received 0.1 mL of PBS. Orbital blood collection and serum collection were performed in all rats before administration and at 4 h, 24 h, 48 h, 72 h, 96 h, and 120 h after administration. After completion of blood collection at 120 h, the rats were sacrificed. The concentration of T4 in the serum of each group of rats was detected using a total T4 ELISA kit (Cat. No.: EIAT4C, ThermoFisher) and a free T4 ELISA kit (Cat. No.: CSB-E05079r, Cusabio).

As shown in FIGs. 3A and 3B and Table 8, CB16-hu3-IgG1 exhibited significant inhibition on serum total T4 and free T4 from 24 h after administration, and the inhibitory effect persisted until the experimental endpoint at 5 days after administration. Moreover, the inhibitory effect was significantly superior to that of the positive control antibody K1-70. Serum free T4 is an active form of T4 that functions *in vivo*. At 120 h after administration, the serum free T4 level in the K1-70 treatment group had recovered to a level comparable to that in the PBS control group, while the serum free T4 level in the CB16-hu3-IgG1 treatment group remained significantly lower. These results demonstrated that CB16-hu3-IgG1 was capable of blocking the TSHR receptor and inhibiting its downstream T4 secretion, with a relatively strong and long-lasting effect.

Table 8. Results of Wistar rat model

| Experimental group | Total T4 (120 h after administration) | Free T4 (120 h after administration) |
|---|---|---|
| PBS | 34.94±8.38 | 10.87±0.31 |
| K1-70 | 15.11±3.3 | 9.2±0.97 |
| CB16-hu3-IgG1 | 4.12±2.56 | 6.2±0.25 |

**Example 7. Construction and Preparation of Anti-IGF-1R/TSHR Bispecific Antibodies**

In this example, anti-IGF-1R/TSHR bispecific antibodies were constructed.

Illustratively, IG05-hu5-1 and CB16-hu3 were respectively linked to the N-terminus and the C-terminus of the IgG1 Fc region (SEQ ID NO: 16) via $(G_4S)_3$ linkers to construct a bispecific antibody 0031-0024. IG06 and CB 16-hu3 were respectively linked to the N-terminus and the C-terminus of the IgG1 Fc region (SEQ ID NO: 16) via $(G_4Q)_3$ linkers to construct a bispecific antibody 0031-0053. Similarly, 0031-0060 and 0031-0070 were constructed. Their sequences are shown below:

> 0031-0024

EVQLVESGGGLVQPGGSLRLSCAASGFTFG<u>TFGMA</u>WFRQAPGKEREGVS<u>AISAS
SGRTYYADSVKG</u>RFTISRDNSKNTLYLQMNSLRAEDTAVYYCAVR<u>SKQGAGYG
FWYEGRLYGY</u>WGQGTLVTVSS*EPKSSDKTHTCPPCPAPELLGGPSVFLFPPKPKD
TLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQG
NVFSCSVMHEALHNHYTQKSLSLSPG*GGGGSGGGGSGGGGGSEVQLLESGGGLVQ
PGGSLRLSCAGPGYTSS<u>CMG</u>WFRQAPGKERTGVS<u>AIYIGGRGTDYADSVKG</u>RF
TISQDNSKNTLYLQMNSLRAEDTAVYYCWA<u>GIEYDCYSGHWRDWAFSRERGF</u>
WGQGTLVTVSS                                                    SEQ ID NO: 49

> 0031-0053

QVQLVESGGGSVQAGGSLRLSCAASRYIVS<u>TRCLG</u>WVRQAPGNEREEIA<u>TIDTA</u>
<u>GRPTYADSVKG</u>RFTISKDGAKNFLYLQMNSLKPEDTAMYYCAA<u>ASSDNCRYFP</u>
<u>SN</u>LGQGTQVTVSS*EPKSSDKTHTCPPCPAPELLGGPSVFLFPPKPKDTLMISRTPEV*
*TCVVVDVSHEDPEVKFNWYVDGVEVHNAKTKPREEQYNSTYRVVSVLTVLHQDWLN*
*GKEYKCKVSNKALPAPIEKTISKAKGQPREPQVYTLPPSRDELTKNQVSLTCLVKGFY*
*PSDIAVEWESNGQPENNYKTTPPVLDSDGSFFLYSKLTVDKSRWQQGNVFSCSVMH*
*EALHNHYTQKSLSLSPG*GGGGQGGGGQGGGGQEVQLLESGGGLVQPGGSLRLSC
AGPGYTSS<u>CMG</u>WFRQAPGKERTGVS<u>AIYIGGRGTD</u>YADSVKGRFTISQDNSKN
TLYLQMNSLRAEDTAVYYCWA<u>GIEYDCYSGHWRDWAFSRERGF</u>WGQGTLVTV
SS                                                                    SEQ ID NO: 50

> 0031-0060

EVQLLESGGGLVQPGGSLRLSCAGPGYTSS<u>CMG</u>WFRQAPGKERTGVS<u>AIYIGGR</u>
<u>GTDYADSVKG</u>RFTISQDNSKNTLYLQMNSLRAEDTAVYYCWA<u>GIEYDCYSGHW</u>
<u>RDWAFSRERGF</u>WGQGTLVTVSS*EPKSSDKTHTCPPCPAPELLGGPSVFLFPPKPK*
*DTLMISRTPEVTCVVVDVSHEDPEVKFNWYVDGVEVHNAKTKPREEQYNSTYRVVSV*
*LTVLHQDWLNGKEYKCKVSNKALPAPIEKTISKAKGQPREPQVYTLPPSRDELTKNQ*
*VSLTCLVKGFYPSDIAVEWESNGQPENNYKTTPPVLDSDGSFFLYSKLTVDKSRWQQ*
*GNVFSCSVMHEALHNHYTQKSLSLSPG*GGGGSGGGGSGGGGSEVQLVESGGGLV
QPGGSLRLSCAASGFTFG<u>TFGMA</u>WFRQAPGKEREGVS<u>AISASSGRTYY</u>ADSVK
GRFTISRDNSKNTLYLQMNSLRAEDTAVYYCAVR<u>SKQGAGYGFWYEGRLYGY</u>
WGQGTLVTVSS                                                        SEQ ID NO:
51

> 0031-0070

EVQLLESGGGLVQPGGSLRLSCAGPGYTSS<u>CMG</u>WFRQAPGKERTGVS<u>AIYIGGR</u>
<u>GTDYADSVKG</u>RFTISQDNSKNTLYLQMNSLRAEDTAVYYCWA<u>GIEYDCYSGHW</u>
<u>RDWAFSRERGF</u>WGQGTLVTVSS*EPKSSDKTHTCPPCPAPELLGGPSVFLFPPKPK*
*DTLMISRTPEVTCVVVDVSHEDPEVKFNWYVDGVEVHNAKTKPREEQYNSTYRVVSV*
*LTVLHQDWLNGKEYKCKVSNKALPAPIEKTISKAKGQPREPQVYTLPPSRDELTKNQ*
*VSLTCLVKGFYPSDIAVEWESNGQPENNYKTTPPVLDSDGSFFLYSKLTVDKSRWQQ*

*GNVFSCSVMHEALHNHYTQKSLSLSPG*GGGGQGGGGQGGGGQQVQLVESGGGS
VQAGGSLRLSCAASRYIVS<u>TRCLG</u>WVRQAPGNEREEIA<u>TIDTAGRPTYADSVKG</u>
RFTISKDGAKNFLYLQMNSLKPEDTAMYYCAA<u>ASSDNCRYFP</u>SNLGQGTQVTV
SS                                                                    SEQ ID NO: 52

The above bispecific antibody sequences were each cloned into a mammalian expression vector pTT5, and ExpiCHO cells were transfected with the vector for expression, followed by purification. After detection, the target bispecific antibodies were obtained.

**Example 8. Detection of ADCC Effects of Antibodies**

This example was intended to detect the ability of antibodies to mediate effector cell-induced cytotoxic killing of target cells.

HEK293 suspension cells were transiently transfected with a plasmid expressing full-length human TSHR using lipofectamine 2000. After 48 h of culture, the cells were resuspended in a phenol red-free RPMI 1640 medium (Gibco, Cat. No. 11835030) containing 2% fetal bovine serum and 10 ng/mL recombinant human IL-2 (Biolegend, Cat. No. 589102). The HEK293-human TSHR target cells were seeded into 96-well U-bottom plates at 20000 cells/well, and pre-incubated with antibodies at different concentrations for 1 h. Subsequently, 50000 NK92-FcγRIIIA-176V3 effector cells were added to each well, and the cells were incubated in a cell incubator at 37 °C for 5 h. After the cells were centrifuged, the supernatant was collected, and detection was performed by adding a substrate using a cytotoxicity detection kit (Promega, Cat. No. G1780). Readings were taken at 490 nm using a spectraMax M5 microplate reader. The values were calculated according to the following formula, and the intensity of the ADCC activity of the antibodies was compared.

$$\text{Cell killing rate (\%)} = \frac{\text{Reading of experimental group} - \text{Background value of effector cell well} - \text{Background value of target cell well}}{\text{Reading of maximum lysis control well} - \text{Background value of target cell well}} \times 100$$

As shown in FIG. 4, the positive control antibody with enhanced ADCC activity by Fc engineering (the Fc region of 0031-0024 comprising S228D/I332E mutations) exhibited a significant killing effect. In contrast, the killing activity induced by the monoclonal antibody CB16-IgG1 was relatively weak, and no ADCC activity was detected for the bispecific antibody 0031-0024, the monoclonal antibody IG05-hu5-1-IgG1, and the positive control antibodies K1-70, Tepezza, VRDN-001, and PHP1003. These results demonstrated that the bispecific antibody 0031-0024 did not induce ADCC effects, suggesting that it has a low risk of inducing cytotoxic killing *in vivo* and favorable safety.

**Example 9. Synergistic Assay for Inhibition of ERK Phosphorylation (pERK) by Antibodies**

In this example, the ability of an anti-IGF-1R/TSHR bispecific antibody to inhibit pERK signals in HEK293-human TSHR cells was detected.

First, suspension-cultured HEK293 cells were transiently transfected with a human TSHR plasmid using lipofectamine 2000, such that endogenous human IGF-1R was expressed on the cell surface and human TSHR was also overexpressed on the cell surface. A pERK synergistic stimulation assay was performed 24 h after transfection, and the specific experimental procedures were as follows.

Cell counting was performed, and HEK293-human TSHR cells were resuspended in HANKS balanced salt solution at a density of $1.25 \times 10^6$ cells/mL. A volume of 40 $\mu$L of the cell suspension was added to each well of a 96-well U-bottom plate. The anti-IGF-1R/TSHR bispecific antibody to be tested was diluted to the required concentrations with HANKS balanced salt solution, and 10 $\mu$L of the diluted antibody solution was added to each well. After the antibody was mixed well with the cells, the 96-well plate was incubated in a cell incubator at 37 °C for 30 min.

Human IGF1 (Beyotime, Cat. No. P5502) and bovine TSH (Sigma, Cat. No. T8931) were diluted to the required concentrations, and 10 $\mu$L of IGF1, TSH, or both (for co-stimulation) was added to each well. After mixing well, the 96-well plate was placed back into the cell incubator and incubated at 37 °C for 6 min. The final concentrations of IGF1 and TSH in each well were 100 ng/mL and 1000 ng/mL, respectively.

Immediately after the stimulation, the cells were resuspended again, and 6 $\mu$L of the cell suspension (about 5000 cells) was transferred into a white 96-well microplate. Then, 2 $\mu$L of 4$\times$ lysis buffer was added for lysis, and the plate was shaken at 400 rpm at room temperature for 30 min. Thereafter, with reference to the instructions of the PHOSPHO-ERK1/2 (THR202/TYR204) DETECTION KITS (PerkinElmer, Cat. No. 64ERKPEG), 2 $\mu$L of a mixture of Phospho-ERK1/2 d2 antibody and Phospho-ERK1/2 Eu Cryptate antibody was added to each well. The plate was covered with a plate-sealing film and then incubated at room temperature for 4 h. Readings were taken at 665 nm and 620 nm using a Tecan Infinite® 200 PRO microplate reader. The values were calculated according to the following formula, and the ability of the antibodies to inhibit pERK signals under co-stimulation with IGF1 and TSH was compared.

$$\text{Ratio} = \frac{Signal\,665\,nm}{Signal\,620\,nm} \times 10000$$

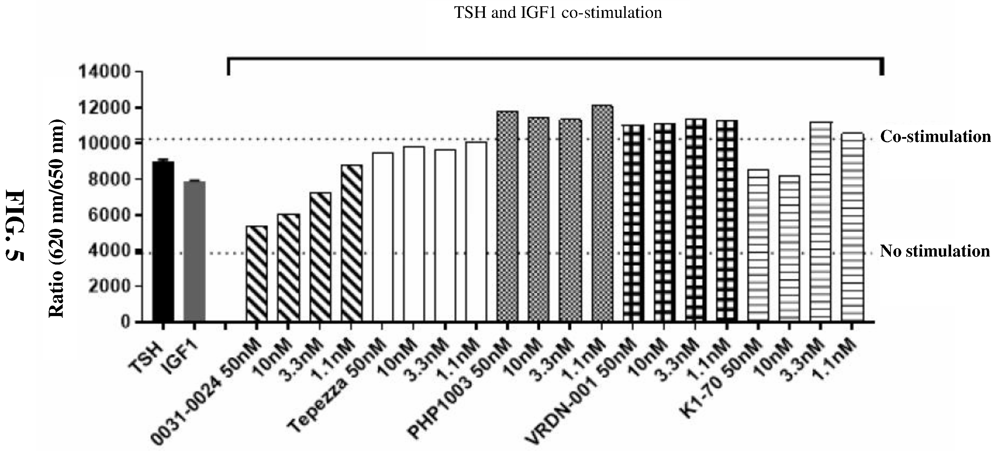

The results in FIG. 5 and Table 9 showed that only the bispecific antibody 0031-0024 produced a significant dose-dependent inhibitory effect on the synergistically induced pERK signals under IGF1/TSH co-stimulation, which was significantly superior to that of other control monoclonal antibodies.

Table 9. Results of inhibition of synergistically induced pERK signals under IGF1/TSH co-stimulation by antibodies

| Experimental group | Ratio of pERK signal values (620 nm/650 nm) |
|---|---|
| 0031-0024 (50 nM) | 5388.054 |
| 0031-0024 (10 nM) | 6046.25 |
| 0031-0024 (3.3 nM) | 7237.161 |
| 0031-0024 (1.1 nM) | 8759.694 |
| Tepezza (50 nM) | / |
| PHP1003 (50 nM) | / |
| VRDN001 (50 nM) | / |
| K1-70 (50 nM) | 8501.972 (with weak pERK inhibition; no dose dependence) |
| K1-70 (10 nM) | 8220.973 (with weak pERK inhibition; no dose dependence) |
| Note: / indicates no ERK phosphorylation inhibitory effect and no dose dependence. | |

## Example 10. Synergistic Assay for Inhibition of Hyaluronic Acid Secretion

Orbital fibroblasts from patients with TAO can secrete hyaluronic acid under stimulation by IGF-1R/TSHR autoantibodies, thereby causing tissue edema. In this example, the ability of IGF-1R/TSHR antibodies to inhibit the secretion of hyaluronic acid (HA) by orbital fibroblasts (OF) of TAO patients was detected.

Retrobulbar adipose connective tissue was obtained from TAO patients undergoing orbital decompression surgery. The obtained surgical specimens were subjected to tissue dissociation to isolate OFs, which were then passaged. The purified cells from the passages were used for the experiment. The specific experimental procedures were as follows: Cell counting was performed, and the OFs were resuspended in a DMEM medium containing 1% fetal bovine serum at a density of $5 \times 10^4$ cells/mL. A volume of 100 $\mu$L of the cell suspension was added to each well of a 96-well plate, and starvation treatment was performed for 24 h. The IGF-1R/TSHR antibodies to be tested were diluted to the required concentrations with a serum-reduced medium, and 50 $\mu$L of the diluted antibody solution was added to each well. The plate was incubated in a cell incubator at 37 °C for 30 min.

Bovine TSH or M22 was diluted to the required concentration, and 50 $\mu$L of bovine TSH was added to each well for stimulation, with the final concentration of bovine TSH in each well being 2 $\mu$g/mL. The plate was then incubated in a cell incubator at 37 °C for 72 h. The level of hyaluronic acid in the culture supernatant was detected using a Hyaluronan Quantikine ELISA Kit (Cat. No.: DHYAL0, R&D Systems).

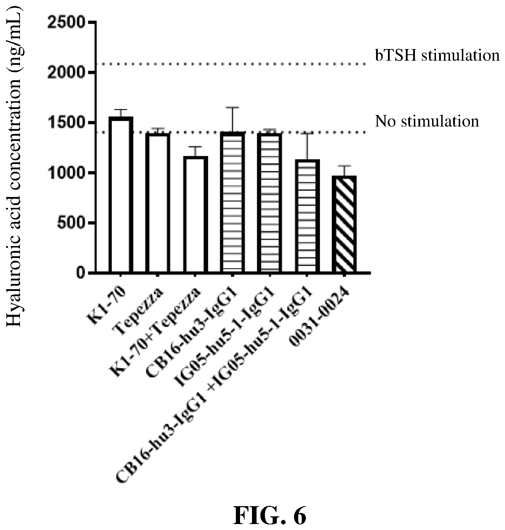

The results in FIG. 6 and Table 10 showed that both TSHR and IGF1R monoclonal antibodies were capable of inhibiting the secretion of HA stimulated by bovine TSH alone; in TAO patient donors, at an antibody concentration of 50 nM, the inhibitory activity of the monoclonal antibody combination and the bispecific antibody 0031-0024 was stronger than that of the control monoclonal antibodies, indicating a trend of synergistic inhibition, and the bispecific antibody 0031-0024 exhibited the strongest inhibitory activity.

Table 10. Results of inhibition of hyaluronic acid production under TSH stimulation by antibodies

| Experimental group (50 nM) | Hyaluronic acid concentration (ng/mL) |
|---|---|
| K1-70 | 1553.74±77.06 |
| Tepezza | 1399.57±43.29 |
| K1-70 + Tepezza | 1170.74±90.55 |
| CB16-hu3-IgG1 | 1404.66±245.14 |
| IG05-hu5-1-IgG1 | 1398.09±32.05 |
| CB16-hu3-IgG1 + IG05-hu5-1-IgG1 | 1139.49±256.05 |
| 0031-0024 | 974.4±96.6 |

**Claims**

1. A TSHR-binding protein, comprising an immunoglobulin single variable domain, wherein the immunoglobulin single variable domain comprises:

   a CDR1, a CDR2, and a CDR3 from the amino acid sequence set forth in any one of SEQ ID NOs: 6 and 31-33, wherein the CDR1, the CDR2, and the CDR3 are numbered according to the Kabat, IMGT, Chothia, AbM, or Contact numbering scheme;
   preferably, the immunoglobulin single variable domain comprises a CDR1, a CDR2, and a CDR3 set forth in SEQ ID NOs: 13, 14, and 15, respectively.

2. The TSHR-binding protein according to claim 1, wherein the immunoglobulin single variable domain undergoes an engineering process selected from the group consisting of any one of the following or a combination thereof: humanization, back mutation, affinity maturation, T cell epitope removal, reduction of antibody deamidation, and reduction of antibody isomerization;
   preferably, heavy chain framework regions of a human germline template used in the humanization engineering process are derived from IGHV3-23 or IGHJ4.

3. The TSHR-binding protein according to claim 1 or 2, wherein the amino acid sequence of the immunoglobulin single variable domain is set forth in any one of SEQ ID NOs: 6 and 31-33 or has at least 80% sequence identity thereto;

   preferably, the TSHR-binding protein is an anti-TSHR antibody or an antigen-binding fragment thereof;
   more preferably, the TSHR-binding protein is an anti-TSHR single-domain antibody or VHH.

4. The TSHR-binding protein according to any one of claims 1 to 3, further comprising an Fc region of an immunoglobulin, wherein

   preferably, the Fc region is an Fc region of human IgG1, human IgG2, human IgG3, or human IgG4;
   more preferably, the Fc region is an Fc region of human IgG1.

5. An IGF-1R/TSHR-binding protein, comprising:

   a first antigen-binding domain that specifically binds to TSHR, and
   a second antigen-binding domain that specifically binds to IGF-1R, wherein
   preferably, the first antigen-binding domain comprises an immunoglobulin single variable domain, and the immunoglobulin single variable domain in the first antigen-binding domain comprises:

   a CDR1, a CDR2, and a CDR3 from the amino acid sequence set forth in any one of SEQ ID NOs: 6 and 31-33, wherein the CDR1, the CDR2, and the CDR3 are numbered according to the Kabat, IMGT, Chothia, AbM, or Contact numbering scheme.

6. The IGF-1R/TSHR-binding protein according to claim 5, wherein the amino acid sequences of the CDR1, the CDR2, and the CDR3 of the immunoglobulin single variable domain in the first antigen-binding domain are set forth in SEQ ID NOs: 13, 14, and 15, respectively.

7. The IGF-1R/TSHR-binding protein according to claim 5 or 6, wherein the second antigen-binding domain comprises an immunoglobulin single variable domain, and the immunoglobulin single variable domain in the second antigen-binding domain comprises:

   a CDR1, a CDR2, and a CDR3 from the amino acid sequence set forth in any one of SEQ ID NOs: 4, 20-24, and 34, or a CDR1, a CDR2, and a CDR3 from the amino acid sequence set forth in any one of SEQ ID NOs: 5 and 25-30, wherein the CDR1, the CDR2, and the CDR3 are numbered according to the Kabat, IMGT, Chothia, AbM, or Contact numbering scheme;
   preferably, the immunoglobulin single variable domain in the second antigen-binding domain comprises:

   a CDR1, a CDR2, and a CDR3 set forth in SEQ ID NOs: 7, 8, and 35, respectively, or
   a CDR1, a CDR2, and a CDR3 set forth in SEQ ID NOs: 10, 11, and 12, respectively;
   more preferably, the immunoglobulin single variable domain in the second antigen-binding domain comprises:

> a CDR1, a CDR2, and a CDR3 set forth in SEQ ID NOs: 7, 8, and 9, respectively, or
> a CDR1, a CDR2, and a CDR3 set forth in SEQ ID NOs: 7, 8, and 36, respectively.

**8.** The IGF-1R/TSHR-binding protein according to any one of claims 5 to 7, wherein the immunoglobulin single variable domain undergoes an engineering process selected from the group consisting of any one of the following or a combination thereof: humanization, back mutation, affinity maturation, T cell epitope removal, reduction of antibody deamidation, and reduction of antibody isomerization;
preferably, heavy chain framework regions of a human germline template used in the humanization engineering process are derived from IGHV3-23, IGHV3-66, and IGHJ4.

**9.** The IGF-1R/TSHR-binding protein according to any one of claims 5 to 8, wherein the amino acid sequence of the immunoglobulin single variable domain in the first antigen-binding domain is set forth in any one of SEQ ID NOs: 6 and 31-33 or has at least 80% sequence identity thereto.

**10.** The IGF-1R/TSHR-binding protein according to any one of claims 5 to 9, wherein the amino acid sequence of the immunoglobulin single variable domain in the second antigen-binding domain is set forth in any one of SEQ ID NOs: 4, 20-24, and 34 or has at least 80% sequence identity thereto, or is set forth in any one of SEQ ID NOs: 5 and 25-30 or has at least 80% sequence identity thereto;
preferably, the immunoglobulin single variable domain in the second antigen-binding domain is an anti-IGF-1R single-domain antibody or VHH.

**11.** The IGF-1R/TSHR-binding protein according to any one of claims 5 to 10, wherein the immunoglobulin single variable domain is a single-domain antibody or VHH.

**12.** The IGF-1R/TSHR-binding protein according to any one of claims 5 to 11, further comprising an Fc region of an immunoglobulin, wherein

> preferably, the Fc region is an Fc region of human IgG1, human IgG2, human IgG3, or human IgG4;
> more preferably, the Fc region is an Fc region of human IgG1;
> most preferably, the Fc region comprises the amino acid sequence set forth in SEQ ID NO: 16 or an amino acid sequence having at least 90% identity thereto.

**13.** The IGF-1R/TSHR-binding protein according to any one of claims 5 to 12, further comprising a linker, wherein

> preferably, the amino acid sequence of the linker is represented by $(G_mS_n)_h$ or $(G_mQ_n)_h$ or $(GGNGT)_h$ or $(YGNGT)_h$, wherein m and n are each independently selected from the group consisting of integers of 1-8, and h is independently selected from the group consisting of integers of 1-20;
> more preferably, the amino acid sequence of the linker is represented by $(G_4Q)_3$ or $(G_4S)_3$.

**14.** The IGF-1R/TSHR-binding protein according to any one of claims 5 to 13, from the amino-terminus to the carboxyl-terminus, being represented by a structure selected from the group consisting of the following:

> [immunoglobulin single variable domain in first antigen-binding domain]-linker 1-Fc region-linker 2-[immunoglobulin single variable domain in second antigen-binding domain], and
> [immunoglobulin single variable domain in second antigen-binding domain]-linker 1-Fc region-linker 2-[immunoglobulin single variable domain in first antigen-binding domain], wherein - is a peptide bond, the linker 1 and the linker 2 may be independently present or absent, and the linker 1 and the linker 2 may be identical or different;
> preferably, the amino acid sequences of the linker 1 and the linker 2 are each independently selected from the group consisting of $(G_mS_n)_h$, $(G_mQ_n)_h$, $(GGNGT)_h$, $(YGNGT)_h$, and $(EPKSS)_h$, wherein m and n are each independently selected from the group consisting of integers of 1-8, and h is independently selected from the group consisting of integers of 1-20;
> more preferably, the linker 1 is absent, and the amino acid sequence of the linker 2 is represented by $(G_4Q)_3$ or $(G_4S)_3$.

**15.** The IGF-1R/TSHR-binding protein according to any one of claims 5 to 14, comprising the amino acid sequence set forth in any one of SEQ ID NOs: 49-52 or an amino acid sequence having at least 80% identity to any one of SEQ ID NOs: 49-52.

16. The IGF-1R/TSHR-binding protein according to any one of claims 5 to 15, being an anti-IGF-1R/TSHR antibody or an antigen-binding fragment thereof.

17. An IGF-1R-binding protein, comprising an immunoglobulin single variable domain, wherein the immunoglobulin single variable domain comprises:

   a CDR1, a CDR2, and a CDR3 from the amino acid sequence set forth in any one of SEQ ID NOs: 4, 20-24, and 34, or a CDR1, a CDR2, and a CDR3 from the amino acid sequence set forth in any one of SEQ ID NOs: 5 and 25-30, wherein the CDR1, the CDR2, and the CDR3 are numbered according to the Kabat, IMGT, Chothia, AbM, or Contact numbering scheme;
   preferably, the immunoglobulin single variable domain comprises:

      a CDR1, a CDR2, and a CDR3 set forth in SEQ ID NOs: 7, 8, and 35, respectively, or
      a CDR1, a CDR2, and a CDR3 set forth in SEQ ID NOs: 10, 11, and 12, respectively;
      more preferably, the immunoglobulin single variable domain comprises:

         a CDR1, a CDR2, and a CDR3 set forth in SEQ ID NOs: 7, 8, and 9, respectively, or
         a CDR1, a CDR2, and a CDR3 set forth in SEQ ID NOs: 7, 8, and 36, respectively.

18. The IGF-1R-binding protein according to claim 17, wherein the immunoglobulin single variable domain undergoes an engineering process selected from the group consisting of any one of the following or a combination thereof: humanization, back mutation, affinity maturation, T cell epitope removal, reduction of antibody deamidation, and reduction of antibody isomerization;
   preferably, heavy chain framework regions of a human germline template used in the humanization engineering process are derived from IGHV3-66 or IGHJ4.

19. The IGF-1R-binding protein according to claim 17 or 18, wherein the amino acid sequence of the immunoglobulin single variable domain is set forth in any one of SEQ ID NOs: 5, 20-24, and 34 or has at least 80% sequence identity thereto, or is set forth in any one of SEQ ID NOs: 4 and 25-30 or has at least 80% sequence identity thereto;

   preferably, the IGF-1R-binding protein is an anti-IGF-1R antibody or an antigen-binding fragment thereof;
   more preferably, the IGF-1R-binding protein is an anti-IGF-1R single-domain antibody or VHH.

20. The IGF-1R-binding protein according to any one of claims 17 to 19, further comprising an Fc region of an immunoglobulin, wherein

   preferably, the Fc region is an Fc region of human IgG1, human IgG2, human IgG3, or human IgG4;
   more preferably, the Fc region is an Fc region of human IgG1.

21. A combination, comprising:

   the TSHR-binding protein according to any one of claims 1 to 4; and
   the IGF-1R-binding protein according to any one of claims 17 to 20.

22. A polynucleotide, wherein the polynucleotide encodes the TSHR-binding protein according to any one of claims 1 to 4, the IGF-1R/TSHR-binding protein according to any one of claims 5 to 16, the IGF-1R-binding protein according to any one of claims 17 to 20, or any combination thereof;
   preferably, the polynucleotide is a DNA or an RNA.

23. A vector, comprising the polynucleotide according to claim 22, wherein
   preferably, the vector expresses the polynucleotide according to claim 22.

24. A host cell, comprising or expressing the polynucleotide according to claim 22 or the vector according to claim 23.

25. A method for preparing a TSHR-binding protein, an IGF-1R/TSHR-binding protein, or an IGF-1R-binding protein, comprising:

expressing the polynucleotide according to claim 22 or the vector according to claim 23 in the host cell according to claim 24, and isolating an expressed TSHR-binding protein,
IGF-1R/TSHR-binding protein, or IGF-IR-binding protein from the host cell, wherein
optionally, the method further comprises a step of purifying the TSHR-binding protein, the IGF-1R/TSHR-binding protein, or the IGF-1R-binding protein.

26. A pharmaceutical composition, comprising:

the TSHR-binding protein according to any one of claims 1 to 4, the IGF-1R/TSHR-binding protein according to any one of claims 5 to 16, the IGF-1R-binding protein according to any one of claims 17 to 20, or any combination thereof; and
at least one pharmaceutically acceptable excipient, diluent, or carrier.

27. A method for treating or preventing a disease, comprising:

administering to a subject in need thereof a therapeutically or prophylactically effective amount of the TSHR-binding protein according to any one of claims 1 to 4, the IGF-1R/TSHR-binding protein according to any one of claims 5 to 16, the IGF-1R-binding protein according to any one of claims 17 to 20, or any combination thereof, or the polynucleotide according to claim 22, the vector according to claim 23, or the pharmaceutical composition according to claim 26, wherein
preferably, the disease is an autoimmune disease;
more preferably, the disease is Graves' orbitopathy.

28. Use of the TSHR-binding protein according to any one of claims 1 to 4, the IGF-1R/TSHR-binding protein according to any one of claims 5 to 16, the IGF-1R-binding protein according to any one of claims 17 to 20, or any combination thereof, or the polynucleotide according to claim 22, the vector according to claim 23, or the pharmaceutical composition according to claim 26 in the manufacture of a medicament for treating or preventing a disease, wherein

preferably, the disease is an autoimmune disease;
more preferably, the disease is Graves' orbitopathy.

29. Use of the TSHR-binding protein according to any one of claims 1 to 4 in combination with the IGF-1R-binding protein according to any one of claims 17 to 20 in the manufacture of a medicament for treating or preventing a disease, wherein

preferably, the disease is an autoimmune disease;
more preferably, the disease is Graves' orbitopathy.

30. A method for treating or preventing a disease, comprising administering to a patient the TSHR-binding protein according to any one of claims 1 to 4 and the IGF-1R-binding protein according to any one of claims 17 to 20, wherein

preferably, the disease is an autoimmune disease;
more preferably, the disease is Graves' orbitopathy.

31. The TSHR-binding protein according to any one of claims 1 to 4 for use in treating or preventing a disease, wherein the TSHR-binding protein is administered in combination with the IGF-1R-binding protein according to any one of claims 17 to 20; preferably, the disease is an autoimmune disease;
more preferably, the disease is Graves' orbitopathy.

**FIG. 1A**

**FIG. 1B**

**FIG. 2A**

**FIG. 2B**

**FIG. 2C**

**FIG. 3A**

**FIG. 3B**

**FIG. 4**

**FIG. 5**

**FIG. 6**

## INTERNATIONAL SEARCH REPORT

| International application No. |
|---|
| **PCT/CN2024/117376** |

| A. | CLASSIFICATION OF SUBJECT MATTER |
|---|---|

C07K 16/28(2006.01)i;  A61K39/395(2006.01)i;  A61P27/02(2006.01)i;  A61P37/06(2006.01)i

According to International Patent Classification (IPC) or to both national classification and IPC

| B. | FIELDS SEARCHED |
|---|---|

Minimum documentation searched (classification system followed by classification symbols)

    IPC:  C07K A61K A61P

Documentation searched other than minimum documentation to the extent that such documents are included in the fields searched

Electronic data base consulted during the international search (name of data base and, where practicable, search terms used)

    CNABS, DWPI, CNTXT, USTXT, EPTXT, WOTXT, JPTXT, CNKI, ISI Web of Science, 万方数据检索系统, Wanfang Data Search System, pubmed, patentics, 中国专利生物序列检索系统, China Patent Biological Sequence Search System, GENBANK, STN: SEQ ID NOs: 1-61, TSHR, 促甲状腺激素受体, thyroid stimulating hormone receptor, thyroid-stimulating hormone receptor, thyrotropin receptor, TSH receptor, IGF-1R, IGF1R, 胰岛素样生长因子-1受体, insulin like growth factor 1 receptor, insulin-like growth factor 1 receptor, insulin-like growth factor-1 receptor, IGF-1 receptor, IGF1 receptor, 抗体, 单抗, antibody, IgG, 单域抗体, 重链抗体, VHH, 北京拓界生物医药科技有限公司, CDR, 格雷夫斯眼病

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 107573419 A (SHENZHEN IN VIVO BIOMEDICINE TECHNOLOGY LIMITED COMPANY) 12 January 2018 (2018-01-12)<br>claims 1-6 and 8-10 | 5, 8, 11-14, 16, 22-28 |
| A | US 2012195897 A1 (DIMITROV, D.S. et al.) 02 August 2012 (2012-08-02)<br>claims 1-3, 5, 7 and 9-23 | 1-31 |
| A | US 2017015749 A1 (NATIONAL RESEARCH COUNCIL OF CANADA) 19 January 2017 (2017-01-19)<br>claims 1, 5, 12-13 and 18 | 1-31 |
| A | US 2022227875 A1 (UNIVERSITY OF FLORIDA RESEARCH FOUNDATION, INCORPORATED) 21 July 2022 (2022-07-21)<br>claims 1, 13, 17, 19 and 22 | 1-31 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 December 2024** | **18 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/117376**

### C.    DOCUMENTS CONSIDERED TO BE RELEVANT

| Category* | Citation of document, with indication, where appropriate, of the relevant passages | Relevant to claim No. |
|---|---|---|
| A | WO 2016064716 A1 (THE UNITED STATES OF AMERICA, AS REPRESENTED BY THE SECRETARY, DEPARTMENT OF HEALTH AND HUMAN SERVICES) 28 April 2016 (2016-04-28)<br>   claims 1-34 | 1-31 |
| A | WO 2022002038 A1 (HARBOUR BIOMED (SHANGHAI) CO., LTD.) 06 January 2022 (2022-01-06)<br>   claims 1-17 | 1-31 |
| A | WO 2023133485 A2 (VIRIDIAN THERAPEUTICS, INC.) 13 July 2023 (2023-07-13)<br>   claims 1-96 | 1-31 |

Form PCT/ISA/210 (second sheet) (July 2022)

## INTERNATIONAL SEARCH REPORT

| | International application No. |
| --- | --- |
| | **PCT/CN2024/117376** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a. ☑ forming part of the international application as filed.

     b. ☐ furnished subsequent to the international filing date for the purposes of international search (Rule 13*ter*.1(a)),

         ☐    accompanied by a statement to the effect that the sequence listing does not go beyond the disclosure in the international application as filed.

2. ☐    With regard to any nucleotide and/or amino acid sequence disclosed in the international application, this report has been established to the extent that a meaningful search could be carried out without a WIPO Standard ST.26 compliant sequence listing.

3. Additional comments:

     The submitted sequence listing lacks the sequence of SEQ ID NO: 13, and the amino acid sequence of SEQ ID NO: 13 in table 1 of the description disclosed in the international application is taken into consideration in the search.

Form PCT/ISA/210 (continuation of first sheet) (July 2022)

**INTERNATIONAL SEARCH REPORT**

| International application No. |
| --- |
| **PCT/CN2024/117376** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **27, 30-31**
   because they relate to subject matter not required to be searched by this Authority, namely:

   Claims 27 and 30-31 respectively relate to a method for treating or preventing diseases, and the use for treating or preventing diseases, which all fall within subject matter for which no search is required by the International Searching Authority as defined in PCT Rule 39.1(iv): (4) methods for treatment of the human or animal body by surgery or therapy, as well as diagnostic methods. The international search is carried out on the basis of the use in the preparation of a corresponding drug.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/117376**

| Patent document cited in search report | | | Publication date (day/month/year) | Patent family member(s) | | | Publication date (day/month/year) |
|---|---|---|---|---|---|---|---|
| CN | 107573419 | A | 12 January 2018 | WO | 2019061562 | A1 | 04 April 2019 |
| US | 2012195897 | A1 | 02 August 2012 | WO | 2011044336 | A2 | 14 April 2011 |
| | | | | WO | 2011044336 | A3 | 17 November 2011 |
| | | | | CA | 2774953 | A1 | 14 April 2011 |
| | | | | EP | 2486055 | A2 | 15 August 2012 |
| | | | | EP | 2486055 | A4 | 17 April 2013 |
| | | | | US | 2015246967 | A1 | 03 September 2015 |
| | | | | US | 9657096 | B2 | 23 May 2017 |
| | | | | AU | 2010303383 | A1 | 19 April 2012 |
| | | | | AU | 2010303383 | B2 | 31 July 2014 |
| | | | | US | 9056907 | B2 | 16 June 2015 |
| US | 2017015749 | A1 | 19 January 2017 | JP | 2017514456 | A | 08 June 2017 |
| | | | | JP | 6541235 | B2 | 10 July 2019 |
| | | | | CA | 2942152 | A1 | 11 September 2015 |
| | | | | CA | 2942152 | C | 01 August 2023 |
| | | | | UA | 120048 | C2 | 25 September 2019 |
| | | | | AU | 2014385799 | A1 | 20 October 2016 |
| | | | | AU | 2014385799 | B2 | 22 October 2020 |
| | | | | CL | 2016002247 | A1 | 24 March 2017 |
| | | | | EA | 201691779 | A1 | 30 January 2017 |
| | | | | EA | 035472 | B1 | 22 June 2020 |
| | | | | NZ | 724866 | A | 29 April 2022 |
| | | | | PE | 20170088 | A1 | 09 March 2017 |
| | | | | US | 10106614 | B2 | 23 October 2018 |
| | | | | ZA | 201606210 | B | 30 January 2019 |
| | | | | BR | 112016020613 | A2 | 23 January 2018 |
| | | | | EP | 3114142 | A1 | 11 January 2017 |
| | | | | EP | 3114142 | A4 | 13 September 2017 |
| | | | | EP | 3114142 | B1 | 05 June 2019 |
| | | | | WO | 2015131256 | A1 | 11 September 2015 |
| | | | | KR | 20160127815 | A | 04 November 2016 |
| | | | | KR | 102355308 | B1 | 24 January 2022 |
| | | | | IL | 247605 | A0 | 30 November 2016 |
| | | | | IL | 247605 | B | 29 August 2019 |
| | | | | DK | 3114142 | T3 | 16 September 2019 |
| | | | | MX | 2016011560 | A | 13 April 2017 |
| US | 2022227875 | A1 | 21 July 2022 | WO | 2020243659 | A1 | 03 December 2020 |
| WO | 2016064716 | A1 | 28 April 2016 | None | | | |
| WO | 2022002038 | A1 | 06 January 2022 | US | 2023303698 | A1 | 28 September 2023 |
| | | | | TW | 202202524 | A | 16 January 2022 |
| | | | | TWI | 806087 | B | 21 June 2023 |
| | | | | EP | 4174092 | A1 | 03 May 2023 |
| | | | | EP | 4174092 | A4 | 20 March 2024 |
| WO | 2023133485 | A2 | 13 July 2023 | EP | 4460524 | A2 | 13 November 2024 |
| | | | | US | 2024360227 | A1 | 31 October 2024 |
| | | | | MX | 2024008530 | A | 26 September 2024 |
| | | | | KR | 20240130134 | A | 28 August 2024 |
| | | | | AU | 2023205915 | A1 | 18 July 2024 |
| | | | | WO | 2023133485 | A3 | 10 August 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311157087
- WO 9404678 A
- US 2022356257 A, Horizon
- WO 2023019171 A, Viridian
- CN 113512116, Suzhou PRO-HEAL

### Non-patent literature cited in the description

- **TERRY J. SMITH**. *N Engl J Med*, 2016, vol. 375, 16
- **WANG Y**. *Invest Ophthalmol Vis Sci*, 2014, vol. 55, 3
- **JADWIGA FURMANIAK**. *Clinical Endocrinology*, 2022, vol. 96, 878-887
- Antibodies: A Laboratory Manual. Cold Spring Harbor Press
- *J. biol. chem.*, 1968, vol. 243, 3558
- **JOHNSON** ; **WU**. *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- AbMTM, A Computer Program for Modeling Variable Regions of Antibodies. **MARTIN et al.** Proc Natl Acad Sci (USA). Oxford Molecular, Ltd., 1989, vol. 86, 9268-9272
- **SAMUDRALA et al.** Ab Initio Protein Structure Prediction Using a Combined Hierarchical Approach. *PROTEINS, Structure, Function and Genetics Suppl.*, 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **HAMERS-CASTERMAN C** ; **ATARHOUCH T** ; **MUYLDERMANS S** ; **ROBINSON G** ; **HAMERS C** ; **SONGA EB** ; **BENDAHMAN N** ; **HAMERS R**. Naturally occurring antibodies devoid of light chains. *Nature*, 1993, vol. 363, 446-448
- **R. VAN DER LINDEN et al.** *Journal of Immunological Methods*, 2000, vol. 240, 185-195
- **LI et al.** *J Biol Chem.*, 2012, vol. 287, 13713-13721
- **DEFFAR et al.** *African Journal of Biotechnology*, 17 June 2009, vol. 8 (12), 2645-2652
- **KABAT, E. A. et al.** Sequences of Proteins of Immunological Interest. 1991
- **MARKS et al.** *Biotechnology*, 1992, vol. 10, 779-783
- **BARBAS et al.** *Proc. Nat. Acad. Sci, USA*, 1994, vol. 91, 3809-3813
- **SHIER et al.** *Gene*, 1995, vol. 169, 147-155
- **YELTON et al.** *Immunol*, 1995, vol. 155, 1994-2004
- **JACKSON et al.** *J. Immunol.*, 1995, vol. 154 (7), 3310-9
- **HAWKINS et al.** *J. Mol. Biol.*, 1992, vol. 226 (3), 889896
- **KS JOHNSON** ; **RE HAWKINS**. Affinity maturation of antibodies using phage display. Oxford University Press, 1996
- **CACECI et al.** *Byte*, 1984, vol. 9, 340-362
- **WONG** ; **LOHMAN**. *Proc. Natl. Acad. Sci. USA*, 1993, vol. 90, 5428-5432
- Antibodies: A Laboratory Manual and Molecular Cloning: A Laboratory Manual. Cold Spring Harbor Laboratory